# EUROPEAN PATENT APPLICATION

(11) **EP 2 853 602 A1**
(43) Date of publication of application: **01.04.2015**
(21) Application number: 14180999.6
(22) Date of filing: 14.08.2014
(51) Int. Cl.: C12R 1/865, C12P 7/06, C12P 7/56

(54) **Yeast cell with increased pyruvate pool in cytosol and method of producing pyruvate-based metabolite using the same**

(30) Priority: 25.09.2013 KR 20130114144
(71) Applicant: Samsung Electronics Co., Ltd, Gyeonggi-do 443-742 (KR)
(72) Inventor: Park, Young-kyoung, 443-803 Gyeonggi-do (KR); Kang, Chang-duk, 443-803 Gyeonggi-do (KR); Song, Ji-yoon, 443-803 Gyeonggi-do (KR); Lee, Ju-young, 443-803 Gyeonggi-do (KR); Lee, Seung-hyun, 443-803 Gyeonggi-do (KR); Cho, Kwang-myung, 433-803 Gyeonggi-do (KR)
(74) Representative: Grünecker, Kinkeldey, Stockmair & Schwanhäusser

(57) **Abstract**

A genetically engineered yeast cell that produces a pyruvate-based metabolite from pyruvate, wherein activity of a mitochondrial pyruvate carrier (MPC) is reduced compared to a parent yeast cell and a method of producing the pyruvate-based metabolite using the yeast cell.

<110>    Samsung Electronics Co., Ltd.

<120>    Yeast cell with increased with puruvate pool in the cytosol and a
         method of producing pyruvate based metabolites using the same

<130>    EP94633FZSEpau

<140>    not yet assigned
<141>    herewith

<150>    10-2013-0114144
<151>    2013-09-25

<160>    55

<170>    KopatentIn 2.0

<210>    1
<211>    130
<212>    PRT
<213>    Saccharomyces cerevisiae

<400>    1
Met Ser Gln Pro Val Gln Arg Ala Ala Ala Arg Ser Phe Leu Gln Lys
  1               5                  10                  15

Tyr Ile Asn Lys Glu Thr Leu Lys Tyr Ile Phe Thr Thr His Phe Trp
             20                  25                  30

Gly Pro Val Ser Asn Phe Gly Ile Pro Ile Ala Ala Ile Tyr Asp Leu
         35                  40                  45

Lys Lys Asp Pro Thr Leu Ile Ser Gly Pro Met Thr Phe Ala Leu Val
     50                  55                  60

Thr Tyr Ser Gly Val Phe Met Lys Tyr Ala Leu Ser Val Ser Pro Lys
 65                  70                  75                  80

Asn Tyr Leu Leu Phe Gly Cys His Leu Ile Asn Glu Thr Ala Gln Leu
                 85                  90                  95

Ala Gln Gly Tyr Arg Phe Leu Lys Tyr Thr Tyr Phe Thr Thr Asp Glu
            100                 105                 110

Glu Lys Lys Ala Leu Asp Lys Glu Trp Lys Glu Lys Glu Lys Thr Gly
        115                 120                 125

Lys Gln
    130


<210>    2
<211>    129
<212>    PRT
<213>     Saccharomyces cerevisiae

<400>    2
Met Ser Thr Ser Ser Val Arg Phe Ala Phe Arg Arg Phe Trp Gln Ser
  1               5                  10                  15

Glu Thr Gly Pro Lys Thr Val His Phe Trp Ala Pro Thr Leu Lys Trp
             20                  25                  30

Gly Leu Val Phe Ala Gly Phe Ser Asp Met Lys Arg Pro Val Glu Lys
            35                  40                  45

Ile Ser Gly Ala Gln Asn Leu Ser Leu Leu Ser Thr Ala Leu Ile Trp
    50                  55                  60

Thr Arg Trp Ser Phe Val Ile Lys Pro Arg Asn Ile Leu Leu Ala Ser
65                  70                  75                  80

Val Asn Ser Phe Leu Cys Leu Thr Ala Gly Tyr Gln Leu Gly Arg Ile
                85                  90                  95

Ala Asn Tyr Arg Ile Arg Asn Gly Asp Ser Ile Ser Gln Leu Cys Ser
            100                 105                 110

Tyr Ile Leu Ser Gly Ala Asp Glu Ser Lys Lys Glu Ile Thr Thr Gly
        115                 120                 125

Arg

<210>    3
<211>    147
<212>    PRT
<213>    Saccharomyces cerevisiae

<400>    3
Met Ser Ala Ser Ala Phe Asn Phe Ala Phe Arg Arg Phe Trp Asn Ser
1               5                   10                  15

Glu Thr Gly Pro Lys Thr Val His Phe Trp Ala Pro Thr Leu Lys Trp
            20                  25                  30

Gly Leu Val Phe Ala Gly Leu Asn Asp Ile Lys Arg Pro Val Glu Lys
            35                  40                  45

Val Ser Gly Ala Gln Asn Leu Ser Leu Leu Ala Thr Ala Leu Ile Trp
    50                  55                  60

Thr Arg Trp Ser Phe Val Ile Lys Pro Lys Asn Tyr Leu Leu Ala Ser
65                  70                  75                  80

Val Asn Phe Phe Leu Gly Cys Thr Ala Gly Tyr His Leu Thr Arg Ile
                85                  90                  95

Ala Asn Phe Arg Ile Arg Asn Gly Asp Ser Phe Lys Gln Val Ile His
            100                 105                 110

Tyr Ile Ile Lys Gly Glu Thr Pro Ala Ala Val Ala Ala Lys Gln Thr
        115                 120                 125

Ala Ser Thr Ser Met Asn Lys Gly Val Ile Gly Thr Asn Pro Pro Ile
    130                 135                 140

Thr His Arg
145

<210>    4
<211>    393
<212>    DNA
<213>    Saccharomyces cerevisiae

<400>    4
atgtctcaac cggttcaacg cgctgcagca cgctcattcc ttcaaaaata catcaataaa    60

gaaactttga aatatatttt cacaacacac ttctggggtc ccgtatcaaa tttcggtatc    120

ccaattgctg ctatatatga tctgaaaaaa gaccctacac taatctctgg cccaatgact    180

tttgctttag ttacctattc aggtgttttc atgaagtatg ctctttcagt atcacccaaa    240

aactacttac tgtttggatg ccaccttatt aatgaaactg cgcaattagc tcaaggctat    300

aggtttctca aatacacgta tttcacaaca gatgaggaga agaaagctct agataaggaa    360

tggaaagaga aagaaaaaac tggtaaacag taa                                 393

<210>    5
<211>    390
<212>    DNA
<213>    Saccharomyces cerevisiae

<400>    5
atgtctacat catccgtacg ttttgcattt aggcggttct ggcaaagtga gacaggcccc    60

aagacggtgc atttctgggc tcctactttg aaatggggtc tggttttcgc tggattcagc    120

gatatgaaga gaccggtgga aaaaatttct ggtgctcaaa atttgtcgct gctatctact    180

gcgctgattt ggactcgttg gtcctttgtc atcaagccaa gaaacatctt gttggcttct    240

gtcaactcgt ttctttgtct gaccgctggc tatcaattgg gtagaattgc caactacagg    300

atacggaatg gcgactctat atcgcaattg tgtagctata ttctcagcgg cgccgacgaa    360

agcaaaaagg aaattactac gggcagataa                                     390

<210>    6
<211>    441
<212>    DNA
<213>    Saccharomyces cerevisiae

<400>    6
atgtcagcat cagcttttaa ttttgccttt agaagatttt ggaatagtga aacaggccct    60

aaaacagtac acttctgggc cccaactttg aagtgggggc tggtcttcgc agggctaaat    120

gatattaaga ggcctgttga gaaggtatca ggagcacaaa atttatcttt attagcgacg    180

gcactgattt ggacgcgttg gtcgtttgtc atcaagccca agaactatct gttagcttcc    240

gtcaattttt tcctgggttg cactgcaggc taccatctaa caagaattgc taactttagg    300

atacggaacg gtgattcttt taaacaggtt attcactaca taataaaagg ggagactcct    360

gcagccgtcg cagcaaagca aactgcatcc acatcgatga acaaaggtgt gatcggtact    420

aatccgccaa taacgcactg a                                              441

<210>    7
<211>    332

<212>   PRT
<213>   Pelodiscus sinensis japonicus

<400>   7
Met Ser Val Lys Glu Leu Leu Ile Gln Asn Val His Lys Glu Glu His
1               5                   10                  15

Ser His Ala His Asn Lys Ile Thr Val Val Gly Val Gly Ala Val Gly
            20                  25                  30

Met Ala Cys Ala Ile Ser Ile Leu Met Lys Asp Leu Ala Asp Glu Leu
        35                  40                  45

Ala Leu Val Asp Val Ile Glu Asp Lys Leu Arg Gly Glu Met Leu Asp
    50                  55                  60

Leu Gln His Gly Ser Leu Phe Leu Arg Thr Pro Lys Ile Val Ser Gly
65                  70                  75                  80

Lys Asp Tyr Ser Val Thr Ala His Ser Lys Leu Val Ile Ile Thr Ala
                85                  90                  95

Gly Ala Arg Gln Gln Glu Gly Glu Ser Arg Leu Asn Leu Val Gln Arg
            100                 105                 110

Asn Val Asn Ile Phe Lys Phe Ile Ile Pro Asn Val Val Lys Tyr Ser
        115                 120                 125

Pro Asp Cys Met Leu Leu Val Val Ser Asn Pro Val Asp Ile Leu Thr
    130                 135                 140

Tyr Val Ala Trp Lys Ile Ser Gly Phe Pro Lys His Arg Val Ile Gly
145                 150                 155                 160

Ser Gly Cys Asn Leu Asp Ser Ala Arg Phe Arg Tyr Leu Met Gly Glu
                165                 170                 175

Lys Leu Gly Ile His Ser Leu Ser Cys His Gly Trp Ile Ile Gly Glu
            180                 185                 190

His Gly Asp Ser Ser Val Pro Val Trp Ser Gly Val Asn Val Ala Gly
        195                 200                 205

Val Ser Leu Lys Ala Leu Tyr Pro Asp Leu Gly Thr Asp Ala Asp Lys
    210                 215                 220

Glu His Trp Lys Glu Val His Lys Gln Val Val Asp Ser Ala Tyr Glu
225                 230                 235                 240

Val Ile Lys Leu Lys Gly Tyr Thr Ser Trp Ala Ile Gly Leu Ser Val
                245                 250                 255

Ala Asp Leu Ala Glu Thr Val Met Lys Asn Leu Arg Arg Val His Pro
            260                 265                 270

Ile Ser Thr Met Val Lys Gly Met Tyr Gly Val Ser Ser Asp Val Phe
        275                 280                 285

Leu Ser Val Pro Cys Val Leu Gly Tyr Ala Gly Ile Thr Asp Val Val
    290                 295                 300

Lys Met Thr Leu Lys Ser Glu Glu Glu Glu Lys Leu Arg Lys Ser Ala
305                 310                 315                 320

Asp Thr Leu Trp Gly Ile Gln Lys Glu Leu Gln Phe
                325                 330

<210>    8
<211>    332
<212>    PRT
<213>    Ornithorhynchus anatinus

<400>    8
Met Ala Gly Val Lys Glu Gln Leu Ile Gln Asn Leu Leu Lys Glu Glu
1               5                   10                  15

Tyr Ala Pro Gln Asn Lys Ile Thr Val Val Gly Val Gly Ala Val Gly
            20                  25                  30

Met Ala Cys Ala Ile Ser Ile Leu Met Lys Asp Leu Ala Asp Glu Leu
        35                  40                  45

Ala Leu Val Asp Val Ile Glu Asp Lys Leu Lys Gly Glu Met Met Asp
    50                  55                  60

Leu Gln His Gly Ser Leu Phe Leu Arg Thr Pro Lys Ile Val Ser Gly
65                  70                  75                  80

Lys Asp Tyr Ser Val Thr Ala Asn Ser Lys Leu Val Ile Ile Thr Ala
                85                  90                  95

Gly Ala Arg Gln Gln Glu Gly Glu Ser Arg Leu Asn Leu Val Gln Arg
            100                 105                 110

Asn Val Asn Ile Phe Lys Phe Ile Ile Pro Asn Val Val Lys Tyr Ser
        115                 120                 125

Pro Asn Cys Lys Leu Leu Val Val Ser Asn Pro Val Asp Ile Leu Thr
    130                 135                 140

Tyr Val Ala Trp Lys Ile Ser Gly Phe Pro Lys Asn Arg Val Ile Gly
145                 150                 155                 160

Ser Gly Cys Asn Leu Asp Ser Ala Arg Phe Arg Tyr Leu Met Gly Glu
                165                 170                 175

Arg Leu Gly Ile His Ser Thr Ser Cys His Gly Trp Val Ile Gly Glu
            180                 185                 190

His Gly Asp Ser Ser Val Pro Val Trp Ser Gly Val Asn Val Ala Gly
        195                 200                 205

Val Ser Leu Lys Asn Leu His Pro Asp Leu Gly Thr Asp Ala Asp Lys
    210                 215                 220

Glu Gln Trp Lys Asp Val His Lys Gln Val Val Asp Ser Ala Tyr Glu
225                 230                 235                 240

Val Ile Lys Leu Lys Gly Tyr Thr Ser Trp Ala Ile Gly Leu Ser Val
                245                 250                 255

Ala Asp Leu Ala Glu Ser Ile Val Lys Asn Leu Arg Arg Val His Pro
            260                 265                 270

Ile Ser Thr Met Ile Lys Gly Leu Tyr Gly Ile Lys Asp Glu Val Phe

275                 280                 285

Leu Ser Val Pro Cys Val Leu Gly Gln Asn Gly Ile Ser Asp Val Val
    290                 295                 300

Lys Ile Thr Leu Lys Ser Glu Glu Glu Ala His Leu Lys Lys Ser Ala
305                 310                 315                 320

Asp Thr Leu Trp Gly Ile Gln Lys Glu Leu Gln Phe
                325                 330


<210>    9
<211>    332
<212>    PRT
<213>    Tursiops truncatus

<400>    9
Met Ala Thr Val Lys Asp Gln Leu Ile Gln Asn Leu Leu Lys Glu Glu
  1               5                  10                  15

His Val Pro Gln Asn Lys Ile Thr Val Val Gly Val Gly Ala Val Gly
             20                  25                  30

Met Ala Cys Ala Ile Ser Ile Leu Met Lys Asp Leu Ala Asp Glu Leu
         35                  40                  45

Ala Leu Val Asp Val Ile Glu Asp Lys Leu Lys Gly Glu Met Met Asp
     50                  55                  60

Leu Gln His Gly Ser Leu Phe Leu Arg Thr Pro Lys Ile Val Ser Gly
 65                  70                  75                  80

Lys Asp Tyr Ser Val Thr Ala Asn Ser Lys Leu Val Ile Ile Thr Ala
                 85                  90                  95

Gly Ala Arg Gln Gln Glu Gly Glu Ser Arg Leu Asn Leu Val Gln Arg
            100                 105                 110

Asn Val Asn Ile Phe Lys Phe Ile Val Pro Asn Ile Val Lys Tyr Ser
        115                 120                 125

Pro His Cys Lys Leu Leu Val Val Ser Asn Pro Val Asp Ile Leu Thr
    130                 135                 140

Tyr Val Ala Trp Lys Ile Ser Gly Phe Pro Lys Asn Arg Val Ile Gly
145                 150                 155                 160

Ser Gly Cys Asn Leu Asp Ser Ala Arg Phe Arg Tyr Leu Met Gly Glu
                165                 170                 175

Arg Leu Gly Val His Pro Leu Ser Cys His Gly Trp Ile Leu Gly Glu
            180                 185                 190

His Gly Asp Ser Ser Val Pro Val Trp Ser Gly Val Asn Val Ala Gly
        195                 200                 205

Val Ser Leu Lys Asn Leu His Pro Glu Leu Gly Thr Asp Ala Asp Lys
    210                 215                 220

Glu His Trp Lys Ala Ile His Lys Gln Val Val Asp Ser Ala Tyr Glu
225                 230                 235                 240

Val Ile Lys Leu Lys Gly Tyr Thr Ser Trp Ala Val Gly Leu Ser Val
                245                 250                 255

Ala Asp Leu Ala Glu Ser Ile Met Lys Asn Leu Arg Arg Val His Pro
            260                 265                 270

Ile Ser Thr Met Ile Lys Gly Leu Tyr Gly Ile Lys Glu Asp Val Phe
        275                 280                 285

Leu Ser Val Pro Cys Ile Leu Gly Gln Asn Gly Ile Ser Asp Val Val
    290                 295                 300

Lys Val Thr Leu Thr Pro Glu Glu Gln Ala Cys Leu Lys Lys Ser Ala
305                 310                 315                 320

Asp Thr Leu Trp Gly Ile Gln Lys Glu Leu Gln Phe
                325                 330

<210>    10
<211>    332
<212>    PRT
<213>    Rattus norvegicus

<400>    10
Met Ala Ala Leu Lys Asp Gln Leu Ile Val Asn Leu Leu Lys Glu Glu
 1               5                  10                  15

Gln Val Pro Gln Asn Lys Ile Thr Val Val Gly Val Gly Ala Val Gly
            20                  25                  30

Met Ala Cys Ala Ile Ser Ile Leu Met Lys Asp Leu Ala Asp Glu Leu
        35                  40                  45

Ala Leu Val Asp Val Ile Glu Asp Lys Leu Lys Gly Glu Met Met Asp
    50                  55                  60

Leu Gln His Gly Ser Leu Phe Leu Lys Thr Pro Lys Ile Val Ser Ser
65                  70                  75                  80

Lys Asp Tyr Ser Val Thr Ala Asn Ser Lys Leu Val Ile Ile Thr Ala
                85                  90                  95

Gly Ala Arg Gln Gln Glu Gly Glu Ser Arg Leu Asn Leu Val Gln Arg
            100                 105                 110

Asn Val Asn Ile Phe Lys Phe Ile Ile Pro Asn Val Val Lys Tyr Ser
        115                 120                 125

Pro Gln Cys Lys Leu Leu Ile Val Ser Asn Pro Val Asp Ile Leu Thr
    130                 135                 140

Tyr Val Ala Trp Lys Ile Ser Gly Phe Pro Lys Asn Arg Val Ile Gly
145                 150                 155                 160

Ser Gly Cys Asn Leu Asp Ser Ala Arg Phe Arg Tyr Leu Met Gly Glu
                165                 170                 175

Arg Leu Gly Val His Pro Leu Ser Cys His Gly Trp Val Leu Gly Glu
            180                 185                 190

His Gly Asp Ser Ser Val Pro Val Trp Ser Gly Val Asn Val Ala Gly
        195                 200                 205

Val Ser Leu Lys Ser Leu Asn Pro Gln Leu Gly Thr Asp Ala Asp Lys
    210                 215                 220

Glu Gln Trp Lys Asp Val His Lys Gln Val Val Asp Ser Ala Tyr Glu
225                 230                 235                 240

Val Ile Lys Leu Lys Gly Tyr Thr Ser Trp Ala Ile Gly Leu Ser Val
                245                 250                 255

Ala Asp Leu Ala Glu Ser Ile Met Lys Asn Leu Arg Arg Val His Pro
            260                 265                 270

Ile Ser Thr Met Ile Lys Gly Leu Tyr Gly Ile Lys Glu Asp Val Phe
        275                 280                 285

Leu Ser Val Pro Cys Ile Leu Gly Gln Asn Gly Ile Ser Asp Val Val
    290                 295                 300

Lys Val Thr Leu Thr Pro Asp Glu Glu Ala Arg Leu Lys Lys Ser Ala
305                 310                 315                 320

Asp Thr Leu Trp Gly Ile Gln Lys Glu Leu Gln Phe
                325                 330


<210>    11
<211>    999
<212>    DNA
<213>    Pelodiscus sinensis japonicus

<400>    11
atgtccgtaa aggaactact tatacaaaac gtccataagg aggagcattc tcacgctcac      60

aataagataa cagttgtagg agtaggtgca gtaggtatgg catgtgctat ttcgatatta     120

atgaaagact tggctgatga actagccttg gttgatgtga ttgaggataa gttacgtgga     180

gaaatgttag atttgcaaca tggttcattg ttcttgagaa cccccaaaat tgtctcgggt     240

aaggattatt cagtcactgc tcattctaaa ctggttatca ttacagcagg tgcaagacag     300

caagaagggg agagcagact aaatctggtt caacgtaatg tcaacatctt caagtttatc     360

atcccgaacg tagtaaaata cagtccagac tgcatgttgc ttgttgtgag taatccagtt     420

gacatcttaa cctatgttgc gtggaaaatc agtgggtttc caaaacatag ggtgattggc     480

tcaggatgca accttgatag cgccaggttt aggtatctaa tgggagaaaa attaggtatt     540

cactccttat cttgtcatgg ctggataata ggcgaacatg gtgattcttc ggtacctgtt     600

tggtccgggg ttaatgtggc tggtgttagt ttaaaagcat tatatcctga cctgggtact     660

gatgccgata aagaacattg gaaagaagtg cacaaacaag tggttgattc tgcttacgaa     720

gttattaaac ttaagggcta cacttcttgg gctataggtc tatcagtagc tgatttggca     780

gaaaccgtta tgaaaaattt aagaagagtc cacccaattt ccacgatggt caagggtatg     840

tacggtgtta gctctgacgt cttcttatct gttccttgtg ttttgggata tgcgggaatt     900

acagacgtcg tgaagatgac attgaaatca gaggaagagg aaaaactaag aaagtcagcc     960

gatactctgt ggggcattca aaaggaattg cagttttaa 999

<210> 12
<211> 1267
<212> DNA
<213> Artificial Sequence

<220>
<223> ARS/CEN

<400> 12
gagctccttt catttctgat aaaagtaaga ttactccatt tatcttttca ccaacatatt 60

catagttgaa agttatcctt ctaagtacgt atacaatatt aattaaacgt aaaaacaaaa 120

ctgactgtaa aaatgtgtaa aaaaaaaata tcaaattcat agcagtttca aggaatgaaa 180

actattatga tctggtcacg tgtatataaa ttattaattt taaacccata taatttatta 240

tttttttatt ctaaagttta aagtaatttt agtagtattt tatattttga ataaatatac 300

tttaaatttt tatttttata ttttattact tttaaaaata atgtttttat ttaaaacaaa 360

attataagtt aaaaagttgt tccgaaagta aaatatattt tatagttttt acaaaaataa 420

attattttta acgtattttt tttaattata tttttgtatg tgattatatc cacaggtatt 480

atgctgaatt tagctgtttc agtttaccag tgtgatagta tgattttttt tgcctctcaa 540

aagctatttt tttagaagct tcgtcttaga aataggtggt gtataaattg cggttgactt 600

ttaactatat atcattttcg atttatttat tacatagaga ggtgctttta attttttaat 660

ttttattttc aataatttta aaagtgggta cttttaaatt ggaacaaagt gaaaaatatc 720

tgttatacgt gcaactgaat tttactgacc ttaaaggact atctcaatcc tggttcagaa 780

atccttgaaa tgattgatat gttggtggat tttctctgat tttcaaacaa gaggtatttt 840

atttcatatt tattatattt tttacattta ttttatattt ttttattgtt tggaagggaa 900

agcgacaatc aaattcaaaa tatattaatt aaactgtaat acttaataag agacaaataa 960

cagccaagaa tcaaatactg ggtttttaat caaaagatct ctctacatgc acccaaattc 1020

attatttaaa tttactatac tacagacaga atatacgaac ccagattaag tagtcagacg 1080

cttttccgct ttattgagta tatagcctta catattttct gcccataatt tctggattta 1140

aaataaacaa aaatggttac tttgtagtta tgaaaaaagg cttttccaaa atgcgaaata 1200

cgtgttattt aaggttaatc aacaaaacgc atatccatat gggtagttgg acaaaacttc 1260

aatcgat 1267

<210> 13
<211> 289
<212> DNA
<213> Artificial Sequence

<220>
<223>    CYC promoter


<400>    13
atttggcgag cgttggttgg tggatcaagc ccacgcgtag gcaatcctcg agcagatccg      60

ccaggcgtgt atatatagcg tggatggcca ggcaacttta gtgctgacac atacaggcat     120

atatatatgt gtgcgacgac acatgatcat atggcatgca tgtgctctgt atgtatataa     180

aactcttgtt ttcttctttt ctctaaatat tctttcctta tacattagga cctttgcagc     240

ataaattact atacttctat agacacgcaa acacaaatac acacactaa                 289


<210>    14
<211>    401
<212>    DNA
<213>    Artificial Sequence

<220>
<223>    TEF promoter


<400>    14
atagcttcaa aatgtttcta ctcctttttt actcttccag attttctcgg actccgcgca      60

tcgccgtacc acttcaaaac acccaagcac agcatactaa atttcccctc tttcttcctc     120

tagggtgtcg ttaattaccc gtactaaagg tttggaaaag aaaaaagaga ccgcctcgtt     180

tctttttctt cgtcgaaaaa ggcaataaaa atttttatca cgtttctttt tcttgaaaat     240

tttttttttg atttttttct ctttcgatga cctcccattg atatttaagt taataaacgg     300

tcttcaattt ctcaagtttc agtttcattt ttcttgttct attacaactt tttttacttc     360

ttgctcatta gaaagaaagc atagcaatct aatctaagtt t                         401


<210>    15
<211>    655
<212>    DNA
<213>    Artificial Sequence

<220>
<223>    GPD promoter


<400>    15
agtttatcat tatcaatact cgccatttca aagaatacgt aaataattaa tagtagtgat      60

tttcctaact ttatttagtc aaaaaattag ccttttaatt ctgctgtaac ccgtacatgc     120

ccaaaatagg gggcgggtta cacagaatat ataacatcgt aggtgtctgg gtgaacagtt     180

tattcctggc atccactaaa tataatggag cccgcttttt aagctggcat ccagaaaaaa     240

aaagaatccc agcaccaaaa tattgttttc ttcaccaacc atcagttcat aggtccattc     300

tcttagcgca actacagaga acaggggcac aaacaggcaa aaaacgggca caacctcaat     360

ggagtgatgc aacctgcctg gagtaaatga tgacacaagg caattgaccc acgcatgtat 420

ctatctcatt ttcttacacc ttctattacc ttctgctctc tctgatttgg aaaaagctga 480

aaaaaaaggt tgaaaccagt tccctgaaat tattccccta cttgactaat aagtatataa 540

agacggtagg tattgattgt aattctgtaa atctatttct taaacttctt aaattctact 600

tttatagtta gtcttttttt tagttttaaa acaccagaac ttagtttcga cggat 655

<210> 16
<211> 1468
<212> DNA
<213> Artificial Sequence

<220>
<223> ADH promoter

<400> 16
gccgggatcg aagaaatgat ggtaaatgaa ataggaaatc aaggagcatg aaggcaaaag 60

acaaatataa gggtcgaacg aaaaataaag tgaaaagtgt tgatatgatg tatttggctt 120

tgcggcgccg aaaaaacgag tttacgcaat tgcacaatca tgctgactct gtggcggacc 180

cgcgctcttg ccggcccggc gataacgctg ggcgtgaggc tgtgcccggc ggagtttttt 240

gcgcctgcat tttccaaggt ttaccctgcg ctaaggggcg agattggaga agcaataaga 300

atgccggttg gggttgcgat gatgacgacc acgacaactg gtgtcattat ttaagttgcc 360

gaaagaacct gagtgcattt gcaacatgag tatactagaa gaatgagcca agacttgcga 420

gacgcgagtt tgccggtggt gcgaacaata gagcgaccat gaccttgaag gtgagacgcg 480

cataaccgct agagtacttt gaagaggaaa cagcaatagg gttgctacca gtataaatag 540

acaggtacat acaacactgg aaatggttgt ctgtttgagt acgctttcaa ttcatttggg 600

tgtgcacttt attatgttac aatatggaag ggaactttac acttctccta tgcacatata 660

ttaattaaag tccaatgcta gtagagaagg ggggtaacac ccctccgcgc tcttttccga 720

tttttttcta aaccgtggaa tatttcggat atccttttgt tgtttccggg tgtacaatat 780

ggacttcctc ttttctggca accaaaccca tacatcggga ttcctataat accttcgttg 840

gtctccctaa catgtaggtg gcggagggga gatatacaat agaacagata ccagacaaga 900

cataatgggc taaacaagac tacaccaatt acactgcctc attgatggtg gtacataacg 960

aactaatact gtagccctag acttgatagc catcatcata tcgaagtttc actacccttt 1020

ttccatttgc catctattga agtaataata ggcgcatgca acttcttttc tttttttttc 1080

ttttctctct cccccgttgt tgtctcacca tatccgcaat gacaaaaaaa tgatggaaga 1140

cactaaagga aaaaattaac gacaaagaca gcaccaacag atgtcgttgt tccagagctg 1200

atgaggggta tctcgaagca cacgaaactt tttccttcct tcattcacgc acactactct 1260

ctaatgagca acggtatacg gccttccttc cagttacttg aatttgaaat aaaaaaaagt 1320

ttgctgtctt gctatcaagt ataaatagac ctgcaattat taatcttttg tttcctcgtc 1380

attgttctcg ttccctttct tccttgtttc tttttctgca caatatttca agctatacca 1440

agcatacaat caactccaag ctggccgc 1468

<210> 17
<211> 252
<212> DNA
<213> Artificial Sequence

<220>
<223> CYC1 terminator

<400> 17
tcatgtaatt agttatgtca cgcttacatt cacgccctcc ccccacatcc gctctaaccg 60

aaaaggaagg agttagacaa cctgaagtct aggtccctat ttattttttt atagttatgt 120

tagtattaag aacgttattt atatttcaaa tttttctttt ttttctgtac agacgcgtgt 180

acgcatgtaa cattatactg aaaaccttgc ttgagaaggt tttgggacgc tcgaaggctt 240

taatttgcgg cc 252

<210> 18
<211> 563
<212> PRT
<213> Saccharomyces cerevisiae

<400> 18
Met Ser Glu Ile Thr Leu Gly Lys Tyr Leu Phe Glu Arg Leu Lys Gln
1               5                   10                  15

Val Asn Val Asn Thr Val Phe Gly Leu Pro Gly Asp Phe Asn Leu Ser
            20                  25                  30

Leu Leu Asp Lys Ile Tyr Glu Val Glu Gly Met Arg Trp Ala Gly Asn
        35                  40                  45

Ala Asn Glu Leu Asn Ala Ala Tyr Ala Ala Asp Gly Tyr Ala Arg Ile
    50                  55                  60

Lys Gly Met Ser Cys Ile Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Val Val Gly Val Pro Ser Ile Ser Ala Gln Ala Lys Gln Leu Leu
            100                 105                 110

Leu His His Thr Leu Gly Asn Gly Asp Phe Thr Val Phe His Arg Met
        115                 120                 125

Ser Ala Asn Ile Ser Glu Thr Thr Ala Met Ile Thr Asp Ile Ala Thr
    130                 135                 140

Ala Pro Ala Glu Ile Asp Arg Cys Ile Arg Thr Thr Tyr Val Thr Gln
145                 150                 155                 160

Arg Pro Val Tyr Leu Gly Leu Pro Ala Asn Leu Val Asp Leu Asn Val
                165                 170                 175

Pro Ala Lys Leu Leu Gln Thr Pro Ile Asp Met Ser Leu Lys Pro Asn
            180                 185                 190

Asp Ala Glu Ser Glu Lys Glu Val Ile Asp Thr Ile Leu Ala Leu Val
        195                 200                 205

Lys Asp Ala Lys Asn Pro Val Ile Leu Ala Asp Ala Cys Cys Ser Arg
    210                 215                 220

His Asp Val Lys Ala Glu Thr Lys Lys Leu Ile Asp Leu Thr Gln Phe
225                 230                 235                 240

Pro Ala Phe Val Thr Pro Met Gly Lys Gly Ser Ile Asp Glu Gln His
                245                 250                 255

Pro Arg Tyr Gly Gly Val Tyr Val Gly Thr Leu Ser Lys Pro Glu Val
            260                 265                 270

Lys Glu Ala Val Glu Ser Ala Asp Leu Ile Leu Ser Val Gly Ala Leu
        275                 280                 285

Leu Ser Asp Phe Asn Thr Gly Ser Phe Ser Tyr Ser Tyr Lys Thr Lys
    290                 295                 300

Asn Ile Val Glu Phe His Ser Asp His Met Lys Ile Arg Asn Ala Thr
305                 310                 315                 320

Phe Pro Gly Val Gln Met Lys Phe Val Leu Gln Lys Leu Leu Thr Thr
                325                 330                 335

Ile Ala Asp Ala Ala Lys Gly Tyr Lys Pro Val Ala Val Pro Ala Arg
            340                 345                 350

Thr Pro Ala Asn Ala Ala Val Pro Ala Ser Thr Pro Leu Lys Gln Glu
        355                 360                 365

Trp Met Trp Asn Gln Leu Gly Asn Phe Leu Gln Glu Gly Asp Val Val
    370                 375                 380

Ile Ala Glu Thr Gly Thr Ser Ala Phe Gly Ile Asn Gln Thr Thr Phe
385                 390                 395                 400

Pro Asn Asn Thr Tyr Gly Ile Ser Gln Val Leu Trp Gly Ser Ile Gly
                405                 410                 415

Phe Thr Thr Gly Ala Thr Leu Gly Ala Ala Phe Ala Ala Glu Glu Ile
            420                 425                 430

Asp Pro Lys Lys Arg Val Ile Leu Phe Ile Gly Asp Gly Ser Leu Gln
        435                 440                 445

Leu Thr Val Gln Glu Ile Ser Thr Met Ile Arg Trp Gly Leu Lys Pro
    450                 455                 460

Tyr Leu Phe Val Leu Asn Asn Asp Gly Tyr Thr Ile Glu Lys Leu Ile
465                 470                 475                 480

His Gly Pro Lys Ala Gln Tyr Asn Glu Ile Gln Gly Trp Asp His Leu
                485                 490                 495

Ser Leu Leu Pro Thr Phe Gly Ala Lys Asp Tyr Glu Thr His Arg Val
            500                 505                 510

Ala Thr Thr Gly Glu Trp Asp Lys Leu Thr Gln Asp Lys Ser Phe Asn
        515                 520                 525

Asp Asn Ser Lys Ile Arg Met Ile Glu Ile Met Leu Pro Val Phe Asp
    530                 535                 540

Ala Pro Gln Asn Leu Val Glu Gln Ala Lys Leu Thr Ala Ala Thr Asn
545                 550                 555                 560

Ala Lys Gln

<210>    19
<211>    1692
<212>    DNA
<213>    Saccharomyces cerevisiae

<400>    19
atgtctgaaa ttactttggg taaatatttg ttcgaaagat taaagcaagt caacgttaac      60

accgttttcg gtttgccagg tgacttcaac ttgtccttgt tggacaagat ctacgaagtt     120

gaaggtatga gatgggctgg taacgccaac gaattgaacg ctgcttacgc cgctgatggt     180

tacgctcgta tcaagggtat gtcttgtatc atcaccacct tcggtgtcgg tgaattgtct     240

gctttgaacg gtattgccgg ttcttacgct gaacacgtcg gtgttttgca cgttgttggt     300

gtcccatcca tctctgctca agctaagcaa ttgttgttgc accacacctt gggtaacggt     360

gacttcactg ttttccacag aatgtctgcc aacatttctg aaaccactgc tatgatcact     420

gacattgcta ccgccccagc tgaaattgac agatgtatca gaaccactta cgtcacccaa     480

agaccagtct acttaggttt gccagctaac ttggtcgact tgaacgtccc agctaagttg     540

ttgcaaactc caattgacat gtctttgaag ccaaacgatg ctgaatccga aaaggaagtc     600

attgacacca tcttggcttt ggtcaaggat gctaagaacc cagttatctt ggctgatgct     660

tgttgttcca gacacgacgt caaggctgaa actaagaagt tgattgactt gactcaattc     720

ccagctttcg tcaccccaat gggtaagggt tccattgacg aacaacaccc aagatacggt     780

ggtgtttacg tcggtacctt gtccaagcca gaagttaagg aagccgttga atctgctgac     840

ttgattttgt ctgtcggtgc tttgttgtct gatttcaaca ccggttcttt ctcttactct     900

tacaagacca agaacattgt cgaattccac tccgaccaca tgaagatcag aaacgccact     960

ttcccaggtg tccaaatgaa attcgttttg caaaagttgt tgaccactat tgctgacgcc    1020

gctaagggtt acaagccagt tgctgtccca gctagaactc cagctaacgc tgctgtccca    1080

gcttctaccc cattgaagca agaatggatg tggaaccaat tgggtaactt cttgcaagaa    1140

ggtgatgttg tcattgctga aaccggtacc tccgctttcg gtatcaacca aaccactttc 1200

ccaaacaaca cctacggtat ctctcaagtc ttatggggtt ccattggttt caccactggt 1260

gctaccttgg gtgctgcttt cgctgctgaa gaaattgatc caaagaagag agttatctta 1320

ttcattggtg acggttcttt gcaattgact gttcaagaaa tctccaccat gatcagatgg 1380

ggcttgaagc catacttgtt cgtcttgaac aacgatggtt acaccattga aaagttgatt 1440

cacggtccaa aggctcaata caacgaaatt caaggttggg accacctatc cttgttgcca 1500

actttcggtg ctaaggacta tgaaacccac agagtcgcta ccaccggtga atgggacaag 1560

ttgacccaag acaagtcttt caacgacaac tctaagatca gaatgattga aatcatgttg 1620

ccagtcttcg atgctccaca aaacttggtt gaacaagcta agttgactgc tgctaccaac 1680

gctaagcaat aa 1692

<210> 20
<211> 591
<212> PRT
<213> Saccharomyces cerevisiae

<400> 20
Met Leu Lys Tyr Lys Pro Leu Leu Lys Ile Ser Lys Asn Cys Glu Ala
1               5                   10                  15

Ala Ile Leu Arg Ala Ser Lys Thr Arg Leu Asn Thr Ile Arg Ala Tyr
            20                  25                  30

Gly Ser Thr Val Pro Lys Ser Lys Ser Phe Glu Gln Asp Ser Arg Lys
        35                  40                  45

Arg Thr Gln Ser Trp Thr Ala Leu Arg Val Gly Ala Ile Leu Ala Ala
    50                  55                  60

Thr Ser Ser Val Ala Tyr Leu Asn Trp His Asn Gly Gln Ile Asp Asn
65                  70                  75                  80

Glu Pro Lys Leu Asp Met Asn Lys Gln Lys Ile Ser Pro Ala Glu Val
                85                  90                  95

Ala Lys His Asn Lys Pro Asp Asp Cys Trp Val Val Ile Asn Gly Tyr
            100                 105                 110

Val Tyr Asp Leu Thr Arg Phe Leu Pro Asn His Pro Gly Gly Gln Asp
        115                 120                 125

Val Ile Lys Phe Asn Ala Gly Lys Asp Val Thr Ala Ile Phe Glu Pro
    130                 135                 140

Leu His Ala Pro Asn Val Ile Asp Lys Tyr Ile Ala Pro Glu Lys Lys
145                 150                 155                 160

Leu Gly Pro Leu Gln Gly Ser Met Pro Pro Glu Leu Val Cys Pro Pro
                165                 170                 175

Tyr Ala Pro Gly Glu Thr Lys Glu Asp Ile Ala Arg Lys Glu Gln Leu
            180                 185                 190

Lys Ser Leu Leu Pro Pro Leu Asp Asn Ile Ile Asn Leu Tyr Asp Phe
        195                 200                 205

Glu Tyr Leu Ala Ser Gln Thr Leu Thr Lys Gln Ala Trp Ala Tyr Tyr
    210                 215                 220

Ser Ser Gly Ala Asn Asp Glu Val Thr His Arg Glu Asn His Asn Ala
225                 230                 235                 240

Tyr His Arg Ile Phe Phe Lys Pro Lys Ile Leu Val Asp Val Arg Lys
                245                 250                 255

Val Asp Ile Ser Thr Asp Met Leu Gly Ser His Val Asp Val Pro Phe
            260                 265                 270

Tyr Val Ser Ala Thr Ala Leu Cys Lys Leu Gly Asn Pro Leu Glu Gly
        275                 280                 285

Glu Lys Asp Val Ala Arg Gly Cys Gly Gln Gly Val Thr Lys Val Pro
    290                 295                 300

Gln Met Ile Ser Thr Leu Ala Ser Cys Ser Pro Glu Glu Ile Ile Glu
305                 310                 315                 320

Ala Ala Pro Ser Asp Lys Gln Ile Gln Trp Tyr Gln Leu Tyr Val Asn
                325                 330                 335

Ser Asp Arg Lys Ile Thr Asp Asp Leu Val Lys Asn Val Glu Lys Leu
            340                 345                 350

Gly Val Lys Ala Leu Phe Val Thr Val Asp Ala Pro Ser Leu Gly Gln
        355                 360                 365

Arg Glu Lys Asp Met Lys Leu Lys Phe Ser Asn Thr Lys Ala Gly Pro
    370                 375                 380

Lys Ala Met Lys Lys Thr Asn Val Glu Glu Ser Gln Gly Ala Ser Arg
385                 390                 395                 400

Ala Leu Ser Lys Phe Ile Asp Pro Ser Leu Thr Trp Lys Asp Ile Glu
                405                 410                 415

Glu Leu Lys Lys Lys Thr Lys Leu Pro Ile Val Ile Lys Gly Val Gln
            420                 425                 430

Arg Thr Glu Asp Val Ile Lys Ala Ala Glu Ile Gly Val Ser Gly Val
        435                 440                 445

Val Leu Ser Asn His Gly Gly Arg Gln Leu Asp Phe Ser Arg Ala Pro
    450                 455                 460

Ile Glu Val Leu Ala Glu Thr Met Pro Ile Leu Glu Gln Arg Asn Leu
465                 470                 475                 480

Lys Asp Lys Leu Glu Val Phe Val Asp Gly Gly Val Arg Arg Gly Thr
                485                 490                 495

Asp Val Leu Lys Ala Leu Cys Leu Gly Ala Lys Gly Val Gly Leu Gly
            500                 505                 510

Arg Pro Phe Leu Tyr Ala Asn Ser Cys Tyr Gly Arg Asn Gly Val Glu
        515                 520                 525

Lys Ala Ile Glu Ile Leu Arg Asp Glu Ile Glu Met Ser Met Arg Leu
    530                 535                 540

Leu Gly Val Thr Ser Ile Ala Glu Leu Lys Pro Asp Leu Leu Asp Leu
545                 550                 555                 560

Ser Thr Leu Lys Ala Arg Thr Val Gly Val Pro Asn Asp Val Leu Tyr
                565                 570                 575

Asn Glu Val Tyr Glu Gly Pro Thr Leu Thr Glu Phe Glu Asp Ala
            580                 585                 590

<210>    21
<211>    1776
<212>    DNA
<213>    Saccharomyces cerevisiae

<400>    21
atgctaaaat acaaaccttt actaaaaatc tcgaagaact gtgaggctgc tatcctcaga      60

gcgtctaaga ctagattgaa cacaatccgc gcgtacggtt ctaccgttcc aaaatccaag     120

tcgttcgaac aagactcaag aaaacgcaca cagtcatgga ctgccttgag agtcggtgca     180

attctagccg ctactagttc cgtggcgtat ctaaactggc ataatggcca aatagacaac     240

gagccgaaac tggatatgaa taaacaaaag atttcgcccg ctgaagttgc caagcataac     300

aagcccgatg attgttgggt tgtgatcaat ggttacgtat acgacttaac gcgattccta     360

ccaaatcatc caggtgggca ggatgttatc aagtttaacg ccgggaaaga tgtcactgct     420

atttttgaac cactacatgc tcctaatgtc atcgataagt atatagctcc cgagaaaaaa     480

ttgggtcccc ttcaaggatc catgcctcct gaacttgtct gtcctcctta tgctcctggt     540

gaaactaagg aagatatcgc tagaaaagaa caactaaaat cgctgctacc tcctctagat     600

aatattatta acctttacga ctttgaatac ttggcctctc aaactttgac taaacaagcg     660

tgggcctact attcctccgg tgctaacgac gaagttactc acagagaaaa ccataatgct     720

tatcatagga tttttttcaa accaaagatc cttgtagatg tacgcaaagt agacatttca     780

actgacatgt tgggttctca tgtggatgtt cccttctacg tgtctgctac agctttgtgt     840

aaactgggaa accccttaga aggtgaaaaa gatgtcgcca gaggttgtgg ccaaggtgtg     900

acaaaagtcc cacaaatgat atctactttg gcttcatgtt cccctgagga aattattgaa     960

gcagcaccct ctgataaaca aattcaatgg taccaactat atgttaactc tgatagaaag    1020

atcactgatg atttggttaa aaatgtagaa aagctgggtg taaaggcatt atttgtcact    1080

gtggatgctc caagtttagg tcaaagagaa aaagatatga agctgaaatt ttccaataca    1140

aaggctggtc caaaagcgat gaagaaaact aatgtagaag aatctcaagg tgcttcgaga    1200

gcgttatcaa agtttattga cccctctttg acttggaaag atatagaaga gttgaagaaa    1260

aagacaaaac tacctattgt tatcaaaggt gttcaacgta ccgaagatgt tatcaaagca 1320

gcagaaatcg gtgtaagtgg ggtggttcta tccaatcatg gtggtagaca attagatttt 1380

tcaagggctc ccattgaagt cctggctgaa accatgccaa tcctggaaca acgtaacttg 1440

aaggataagt tggaagtttt cgtggacggt ggtgttcgtc gtggtacaga tgtcttgaaa 1500

gcgttatgtc taggtgctaa aggtgttggt ttgggtagac cattcttgta tgcgaactca 1560

tgctatggtc gtaatggtgt tgaaaaagcc attgaaattt taagagatga aattgaaatg 1620

tctatgagac tattaggtgt tactagcatt gcggaattga agcctgatct tttagatcta 1680

tcaacactaa aggcaagaac agttggagta ccaaacgacg tgctgtataa tgaagtttat 1740

gagggaccta ctttaacaga atttgaggat gcatga 1776

<210> 22
<211> 391
<212> PRT
<213> Saccharomyces cerevisiae

<400> 22
Met Ser Ala Ala Ala Asp Arg Leu Asn Leu Thr Ser Gly His Leu Asn
1               5                   10                  15

Ala Gly Arg Lys Arg Ser Ser Ser Ser Val Ser Leu Lys Ala Ala Glu
            20                  25                  30

Lys Pro Phe Lys Val Thr Val Ile Gly Ser Gly Asn Trp Gly Thr Thr
        35                  40                  45

Ile Ala Lys Val Val Ala Glu Asn Cys Lys Gly Tyr Pro Glu Val Phe
    50                  55                  60

Ala Pro Ile Val Gln Met Trp Val Phe Glu Glu Glu Ile Asn Gly Glu
65                  70                  75                  80

Lys Leu Thr Glu Ile Ile Asn Thr Arg His Gln Asn Val Lys Tyr Leu
                85                  90                  95

Pro Gly Ile Thr Leu Pro Asp Asn Leu Val Ala Asn Pro Asp Leu Ile
            100                 105                 110

Asp Ser Val Lys Asp Val Asp Ile Ile Val Phe Asn Ile Pro His Gln
        115                 120                 125

Phe Leu Pro Arg Ile Cys Ser Gln Leu Lys Gly His Val Asp Ser His
    130                 135                 140

Val Arg Ala Ile Ser Cys Leu Lys Gly Phe Glu Val Gly Ala Lys Gly
145                 150                 155                 160

Val Gln Leu Leu Ser Ser Tyr Ile Thr Glu Glu Leu Gly Ile Gln Cys
                165                 170                 175

Gly Ala Leu Ser Gly Ala Asn Ile Ala Thr Glu Val Ala Gln Glu His
            180                 185                 190

Trp Ser Glu Thr Thr Val Ala Tyr His Ile Pro Lys Asp Phe Arg Gly

195                 200                 205

Glu Gly Lys Asp Val Asp His Lys Val Leu Lys Ala Leu Phe His Arg
    210                 215                 220

Pro Tyr Phe His Val Ser Val Ile Glu Asp Val Ala Gly Ile Ser Ile
225                 230                 235                 240

Cys Gly Ala Leu Lys Asn Val Val Ala Leu Gly Cys Gly Phe Val Glu
                245                 250                 255

Gly Leu Gly Trp Gly Asn Asn Ala Ser Ala Ala Ile Gln Arg Val Gly
            260                 265                 270

Leu Gly Glu Ile Ile Arg Phe Gly Gln Met Phe Phe Pro Glu Ser Arg
        275                 280                 285

Glu Glu Thr Tyr Tyr Gln Glu Ser Ala Gly Val Ala Asp Leu Ile Thr
    290                 295                 300

Thr Cys Ala Gly Gly Arg Asn Val Lys Val Ala Arg Leu Met Ala Thr
305                 310                 315                 320

Ser Gly Lys Asp Ala Trp Glu Cys Glu Lys Glu Leu Leu Asn Gly Gln
                325                 330                 335

Ser Ala Gln Gly Leu Ile Thr Cys Lys Glu Val His Glu Trp Leu Glu
            340                 345                 350

Thr Cys Gly Ser Val Glu Asp Phe Pro Leu Phe Glu Ala Val Tyr Gln
        355                 360                 365

Ile Val Tyr Asn Asn Tyr Pro Met Lys Asn Leu Pro Asp Met Ile Glu
    370                 375                 380

Glu Leu Asp Leu His Glu Asp
385                 390

<210>    23
<211>    1176
<212>    DNA
<213>    Saccharomyces cerevisiae

<400>    23
atgtctgctg ctgctgatag attaaactta acttccggcc acttgaatgc tggtagaaag      60

agaagttcct cttctgtttc tttgaaggct gccgaaaagc ctttcaaggt tactgtgatt     120

ggatctggta actggggtac tactattgcc aaggtggttg ccgaaaattg taagggatac     180

ccagaagttt tcgctccaat agtacaaatg tgggtgttcg aagaagagat caatggtgaa     240

aaattgactg aaatcataaa tactagacat caaaacgtga aatacttgcc tggcatcact     300

ctacccgaca atttggttgc taatccagac ttgattgatt cagtcaagga tgtcgacatc     360

atcgttttca acattccaca tcaatttttg ccccgtatct gtagccaatt gaaaggtcat     420

gttgattcac acgtcagagc tatctcctgt ctaaagggtt ttgaagttgg tgctaaaggt     480

gtccaattgc tatcctctta catcactgag gaactaggta ttcaatgtgg tgctctatct     540

ggtgctaaca ttgccaccga agtcgctcaa gaacactggt ctgaaacaac agttgcttac 600

cacattccaa aggatttcag aggcgagggc aaggacgtcg accataaggt tctaaaggcc 660

ttgttccaca gaccttactt ccacgttagt gtcatcgaag atgttgctgg tatctccatc 720

tgtggtgctt tgaagaacgt tgttgcctta ggttgtggtt tcgtcgaagg tctaggctgg 780

ggtaacaacg cttctgctgc catccaaaga gtcggtttgg gtgagatcat cagattcggt 840

caaatgtttt tcccagaatc tagagaagaa acatactacc aagagtctgc tggtgttgct 900

gatttgatca ccacctgcgc tggtggtaga aacgtcaagg ttgctaggct aatggctact 960

tctggtaagg acgcctggga atgtgaaaag gagttgttga atggccaatc cgctcaaggt 1020

ttaattacct gcaaagaagt tcacgaatgg ttggaaacat gtggctctgt cgaagacttc 1080

ccattatttg aagccgtata ccaaatcgtt tacaacaact acccaatgaa gaacctgccg 1140

gacatgattg aagaattaga tctacatgaa gattag 1176

<210> 24
<211> 347
<212> PRT
<213> Saccharomyces cerevisiae

<400> 24
Met Ser Ile Pro Glu Thr Gln Lys Gly Val Ile Phe Tyr Glu Ser His
1               5                   10                  15

Gly Lys Leu Glu Tyr Lys Asp Ile Pro Val Pro Lys Pro Lys Ala Asn
            20                  25                  30

Glu Leu Leu Ile Asn Val Lys Tyr Ser Gly Val Cys His Thr Asp Leu
        35                  40                  45

His Ala Trp His Gly Asp Trp Pro Leu Pro Val Lys Leu Pro Leu Val
    50                  55                  60

Gly Gly His Glu Gly Ala Gly Val Val Val Gly Met Gly Glu Asn Val
65                  70                  75                  80

Lys Gly Trp Lys Ile Gly Asp Tyr Ala Gly Ile Lys Trp Leu Asn Gly
                85                  90                  95

Ser Cys Met Ala Cys Glu Tyr Cys Glu Leu Gly Asn Glu Ser Asn Cys
            100                 105                 110

Pro His Ala Asp Leu Ser Gly Tyr Thr His Asp Gly Ser Phe Gln Gln
        115                 120                 125

Tyr Ala Thr Ala Asp Ala Val Gln Ala Ala His Ile Pro Gln Gly Thr
    130                 135                 140

Asp Leu Ala Gln Val Ala Pro Ile Leu Cys Ala Gly Ile Thr Val Tyr
145                 150                 155                 160

Lys Ala Leu Lys Ser Ala Asn Leu Met Ala Gly His Trp Val Ala Ile
                165                 170                 175

Ser Gly Ala Ala Gly Gly Leu Gly Ser Leu Ala Val Gln Tyr Ala Lys

180                 185                 190

Ala Met Gly Tyr Arg Val Leu Gly Ile Asp Gly Gly Glu Gly Lys Glu
        195                 200                 205

Glu Leu Phe Arg Ser Ile Gly Gly Glu Val Phe Ile Asp Phe Thr Lys
    210                 215                 220

Glu Lys Asp Ile Val Gly Ala Val Leu Lys Ala Thr Asp Gly Gly Ala
225                 230                 235                 240

His Gly Val Ile Asn Val Ser Val Ser Glu Ala Ala Ile Glu Ala Ser
                245                 250                 255

Thr Arg Tyr Val Arg Ala Asn Gly Thr Thr Val Leu Val Gly Met Pro
            260                 265                 270

Ala Gly Ala Lys Cys Cys Ser Asp Val Phe Asn Gln Val Val Lys Ser
        275                 280                 285

Ile Ser Ile Gly Ser Tyr Val Gly Asn Arg Ala Asp Thr Arg Glu Ala
    290                 295                 300

Leu Asp Phe Phe Ala Arg Gly Leu Val Lys Ser Pro Ile Lys Val Val
305                 310                 315                 320

Gly Leu Ser Thr Leu Pro Glu Ile Tyr Glu Lys Met Glu Lys Gly Gln
                325                 330                 335

Ile Val Gly Arg Tyr Val Val Asp Thr Ser Lys
            340                 345

<210>    25
<211>    1047
<212>    DNA
<213>    Saccharomyces cerevisiae

<400>    25
atgtctatcc cagaaactca aaaaggtgtt atcttctacg aatcccacgg taagttggaa      60

tacaaagata ttccagttcc aaagccaaag gccaacgaat tgttgatcaa cgttaaatac     120

tctggtgtct gtcacactga cttgcacgct tggcacggtg actggccatt gccagttaag     180

ctaccattag tcggtggtca cgaaggtgcc ggtgtcgttg tcggcatggg tgaaaacgtt     240

aagggctgga agatcggtga ctacgccggt atcaaatggt tgaacggttc ttgtatggcc     300

tgtgaatact gtgaattggg taacgaatcc aactgtcctc acgctgactt gtctggttac     360

acccacgacg gttctttcca acaatacgct accgctgacg ctgttcaagc cgctcacatt     420

cctcaaggta ccgacttggc ccaagtcgcc cccatcttgt gtgctggtat caccgtctac     480

aaggctttga agtctgctaa cttgatggcc ggtcactggg ttgctatctc cggtgctgct     540

ggtggtctag gttctttggc tgttcaatac gccaaggcta tgggttacag agtcttgggt     600

attgacggtg gtgaaggtaa ggaagaatta ttcagatcca tcggtggtga agtcttcatt     660

gacttcacta aggaaaagga cattgtcggt gctgttctaa aggccactga cggtggtgct     720

cacggtgtca tcaacgtttc cgtttccgaa gccgctattg aagcttctac cagatacgtt 780

agagctaacg gtaccaccgt tttggtcggt atgccagctg gtgccaagtg ttgttctgat 840

gtcttcaacc aagtcgtcaa gtccatctct attgttggtt cttacgtcgg taacagagct 900

gacaccagag aagctttgga cttcttcgcc agaggtttgg tcaagtctcc aatcaaggtt 960

gtcggcttgt ctaccttgcc agaaatttac gaaaagatgg aaaagggtca aatcgttggt 1020

agatacgttg ttgacacttc taaataa 1047

<210> 26
<211> 560
<212> PRT
<213> Saccharomyces cerevisiae

<400> 26
Met Ile Arg Gln Ser Leu Met Lys Thr Val Trp Ala Asn Ser Ser Arg
1               5                   10                  15

Phe Ser Leu Gln Ser Lys Ser Gly Leu Val Lys Tyr Ala Lys Asn Arg
            20                  25                  30

Ser Phe His Ala Ala Arg Asn Leu Leu Glu Asp Lys Lys Val Ile Leu
        35                  40                  45

Gln Lys Val Ala Pro Thr Thr Gly Val Val Ala Lys Gln Ser Phe Phe
    50                  55                  60

Lys Arg Thr Gly Lys Phe Thr Leu Lys Ala Leu Leu Tyr Ser Ala Leu
65                  70                  75                  80

Ala Gly Thr Ala Tyr Val Ser Tyr Ser Leu Tyr Arg Glu Ala Asn Pro
                85                  90                  95

Ser Thr Gln Val Pro Gln Ser Asp Thr Phe Pro Asn Gly Ser Lys Arg
            100                 105                 110

Lys Thr Leu Val Ile Leu Gly Ser Gly Trp Gly Ser Val Ser Leu Leu
        115                 120                 125

Lys Asn Leu Asp Thr Thr Leu Tyr Asn Val Val Val Val Ser Pro Arg
    130                 135                 140

Asn Tyr Phe Leu Phe Thr Pro Leu Leu Pro Ser Thr Pro Val Gly Thr
145                 150                 155                 160

Ile Glu Leu Lys Ser Ile Val Glu Pro Val Arg Thr Ile Ala Arg Arg
                165                 170                 175

Ser His Gly Glu Val His Tyr Tyr Glu Ala Glu Ala Tyr Asp Val Asp
            180                 185                 190

Pro Glu Asn Lys Thr Ile Lys Val Lys Ser Ser Ala Lys Asn Asn Asp
        195                 200                 205

Tyr Asp Leu Asp Leu Lys Tyr Asp Tyr Leu Val Val Gly Val Gly Ala
    210                 215                 220

Gln Pro Asn Thr Phe Gly Thr Pro Gly Val Tyr Glu Tyr Ser Ser Phe
225                 230                 235                 240

Leu Lys Glu Ile Ser Asp Ala Gln Glu Ile Arg Leu Lys Ile Met Ser
                245                 250                 255

Ser Ile Glu Lys Ala Ala Ser Leu Ser Pro Lys Asp Pro Glu Arg Ala
            260                 265                 270

Arg Leu Leu Ser Phe Val Val Val Gly Gly Gly Pro Thr Gly Val Glu
        275                 280                 285

Phe Ala Ala Glu Leu Arg Asp Tyr Val Asp Gln Asp Leu Arg Lys Trp
    290                 295                 300

Met Pro Glu Leu Ser Lys Glu Ile Lys Val Thr Leu Val Glu Ala Leu
305                 310                 315                 320

Pro Asn Ile Leu Asn Met Phe Asp Lys Tyr Leu Val Asp Tyr Ala Gln
                325                 330                 335

Asp Leu Phe Lys Glu Glu Lys Ile Asp Leu Arg Leu Lys Thr Met Val
            340                 345                 350

Lys Lys Val Asp Ala Thr Thr Ile Thr Ala Lys Thr Gly Asp Gly Asp
        355                 360                 365

Ile Glu Asn Ile Pro Tyr Gly Val Leu Val Trp Ala Thr Gly Asn Ala
    370                 375                 380

Pro Arg Glu Val Ser Lys Asn Leu Met Thr Lys Leu Glu Glu Gln Asp
385                 390                 395                 400

Ser Arg Arg Gly Leu Leu Ile Asp Asn Lys Leu Gln Leu Leu Gly Ala
                405                 410                 415

Lys Gly Ser Ile Phe Ala Ile Gly Asp Cys Thr Phe His Pro Gly Leu
            420                 425                 430

Phe Pro Thr Ala Gln Val Ala His Gln Glu Gly Glu Tyr Leu Ala Gln
        435                 440                 445

Tyr Phe Lys Lys Ala Tyr Lys Ile Asp Gln Leu Asn Trp Lys Met Thr
    450                 455                 460

His Ala Lys Asp Asp Ser Glu Val Ala Arg Leu Lys Asn Gln Ile Val
465                 470                 475                 480

Lys Thr Gln Ser Gln Ile Glu Asp Phe Lys Tyr Asn His Lys Gly Ala
                485                 490                 495

Leu Ala Tyr Ile Gly Ser Asp Lys Ala Ile Ala Asp Leu Ala Val Gly
            500                 505                 510

Glu Ala Lys Tyr Arg Leu Ala Gly Ser Phe Thr Phe Leu Phe Trp Lys
        515                 520                 525

Ser Ala Tyr Leu Ala Met Cys Leu Ser Phe Arg Asn Arg Val Leu Val
    530                 535                 540

Ala Met Asp Trp Ala Lys Val Tyr Phe Leu Gly Arg Asp Ser Ser Ile
545                 550                 555                 560

<210>    27

<211>    545
<212>    PRT
<213>    Saccharomyces cerevisiae

<400>    27
Met Leu Pro Arg Leu Gly Phe Ala Arg Thr Ala Arg Ser Ile His Arg
1               5                   10                  15

Phe Lys Met Thr Gln Ile Ser Lys Pro Phe Phe His Ser Thr Glu Val
            20                  25                  30

Gly Lys Pro Gly Pro Gln Gln Lys Leu Ser Lys Ser Tyr Thr Ala Val
        35                  40                  45

Phe Lys Lys Trp Phe Val Arg Gly Leu Lys Leu Thr Phe Tyr Thr Thr
    50                  55                  60

Leu Ala Gly Thr Leu Tyr Val Ser Tyr Glu Leu Tyr Lys Glu Ser Asn
65                  70                  75                  80

Pro Pro Lys Gln Val Pro Gln Ser Thr Ala Phe Ala Asn Gly Leu Lys
                85                  90                  95

Lys Lys Glu Leu Val Ile Leu Gly Thr Gly Trp Gly Ala Ile Ser Leu
            100                 105                 110

Leu Lys Lys Leu Asp Thr Ser Leu Tyr Asn Val Thr Val Val Ser Pro
        115                 120                 125

Arg Ser Phe Phe Leu Phe Thr Pro Leu Leu Pro Ser Thr Pro Val Gly
    130                 135                 140

Thr Ile Glu Met Lys Ser Ile Val Glu Pro Val Arg Ser Ile Ala Arg
145                 150                 155                 160

Arg Thr Pro Gly Glu Val His Tyr Ile Glu Ala Glu Ala Leu Asp Val
                165                 170                 175

Asp Pro Lys Ala Lys Lys Val Met Val Gln Ser Val Ser Glu Asp Glu
            180                 185                 190

Tyr Phe Val Ser Ser Leu Ser Tyr Asp Tyr Leu Val Val Ser Val Gly
        195                 200                 205

Ala Lys Thr Thr Thr Phe Asn Ile Pro Gly Val Tyr Gly Asn Ala Asn
    210                 215                 220

Phe Leu Lys Glu Ile Glu Asp Ala Gln Asn Ile Arg Met Lys Leu Met
225                 230                 235                 240

Lys Thr Ile Glu Gln Ala Ser Ser Phe Pro Val Asn Asp Pro Glu Arg
                245                 250                 255

Lys Arg Leu Leu Thr Phe Val Val Val Gly Gly Gly Pro Thr Gly Val
            260                 265                 270

Glu Phe Ala Ala Glu Leu Gln Asp Tyr Ile Asn Gln Asp Leu Arg Lys
        275                 280                 285

Trp Met Pro Asp Leu Ser Lys Glu Met Lys Val Ile Leu Ile Glu Ala
    290                 295                 300

Leu Pro Asn Ile Leu Asn Met Phe Asp Lys Thr Leu Ile Lys Tyr Ala

305                 310                 315                 320

Glu Asp Leu Phe Ala Arg Asp Glu Ile Asp Leu Gln Val Asn Thr Ala
                325                 330                 335

Val Lys Val Val Glu Pro Thr Tyr Ile Arg Thr Leu Gln Asn Gly Gln
            340                 345                 350

Thr Asn Thr Asp Ile Glu Tyr Gly Met Leu Val Trp Ala Thr Gly Asn
        355                 360                 365

Glu Pro Ile Asp Phe Ser Lys Thr Leu Met Ser Arg Ile Pro Glu Gln
    370                 375                 380

Thr Asn Arg Arg Gly Leu Leu Ile Asn Asp Lys Leu Glu Leu Leu Gly
385                 390                 395                 400

Ser Glu Asn Ser Ile Tyr Ala Ile Gly Asp Cys Thr Ala His Thr Gly
                405                 410                 415

Phe Phe Pro Thr Ala Gln Val Ala His Gln Glu Gly Glu Tyr Leu Ala
            420                 425                 430

Lys Ile Leu Asp Lys Lys Leu Gln Ile Glu Gln Leu Glu Trp Asp Met
        435                 440                 445

Leu Asn Ser Thr Asp Glu Thr Glu Val Ser Arg Leu Gln Lys Glu Val
    450                 455                 460

Asn Leu Arg Lys Ser Lys Leu Asp Lys Phe Asn Tyr Lys His Met Gly
465                 470                 475                 480

Ala Leu Ala Tyr Ile Gly Ser Glu Thr Ala Ile Ala Asp Leu His Met
                485                 490                 495

Gly Asp Ser Ser Tyr Gln Leu Lys Gly Met Phe Ala Phe Leu Phe Trp
            500                 505                 510

Lys Ser Ala Tyr Leu Ala Met Cys Leu Ser Ile Arg Asn Arg Ile Leu
        515                 520                 525

Ile Ala Met Asp Trp Thr Lys Val Tyr Phe Leu Gly Arg Asp Ser Ser
    530                 535                 540

Val
545

<210>    28
<211>    1683
<212>    DNA
<213>    Saccharomyces cerevisiae

<400>    28
atgattagac aatcattaat gaaaacagtg tgggctaact cctccaggtt tagcctacag        60

agcaagtcgg ggcttgtgaa atatgccaaa aatagatcgt tccatgcagc aagaaatttg       120

ctagaggaca agaaagtcat tttgcaaaaa gtggcgccca ctactggcgt tgttgcgaag       180

cagtcctttt tcaagagaac tgggaaattt actttgaagg ctttattgta ttctgccctc       240

gcgggtacgg cttacgtttc atactcactt taccgagaag ctaacccttc tacccaagtt       300

cctcaatcgg acacttttcc aaacggttca aagaggaaga ctttggtaat tctgggctcc 360

ggttggggtt ctgtgtcgct tttgaaaaat ttggacacca cgttgtataa tgttgttgtt 420

gtttctccaa gaaattattt tctttttact ccgctattgc catctacccc agttggtacc 480

atcgaattga aatctattgt tgaacctgtc aggactattg ctagaagatc gcacggtgaa 540

gtccattact atgaagctga agcgtacgac gttgatcctg aaaacaaaac aattaaggtc 600

aaatcttccg ctaagaataa cgactacgac ttggacttga aatacgacta tctggttgtc 660

ggtgtgggtg ctcaaccaaa cacttttggt actccgggag tttatgaata ttcttctttc 720

ttgaaggaaa tatccgacgc tcaagagatc agattaaaaa ttatgtccag tattgagaaa 780

gctgcctccc tatctccaaa agatcctgag agagcaagat tgttgagctt tgttgtcgtt 840

ggtggtggtc ccaccggtgt cgaatttgcc gctgaattga gagattatgt tgaccaggac 900

ttgagaaaat ggatgcccga attgagtaaa gaaattaaag tcactttggt ggaggctttg 960

ccaaacattt tgaacatgtt tgacaagtat ctcgttgact atgctcaaga tttattcaaa 1020

gaggaaaaaa tcgatttaag attgaaaaca atggttaaga aagttgacgc taccactata 1080

actgccaaaa ctggcgatgg tgacattgaa aatataccgt atggtgtatt agtttgggct 1140

acaggtaatg cgccaagaga agtgtctaag aacctaatga ctaaattaga ggaacaggac 1200

tcaagacgtg gtttgttgat agataacaaa cttcaacttt tgggtgctaa gggatctatt 1260

tttgctatcg gcgattgtac cttccaccct ggcttgttcc ctaccgctca agttgcccac 1320

caagaaggtg aatacttggc tcagtatttc aagaaagctt ataaaatcga tcaattgaac 1380

tggaaaatga cccatgctaa agacgattca gaagtcgcta gattaaagaa ccaaatagtc 1440

aaaacgcaat cgcaaattga agacttcaag tacaaccata agggtgctct ggcttatatt 1500

ggttcagata aagccattgc tgatcttgcc gttggtgaag ccaaatatag gttagccggc 1560

tcattcacct tcctattctg gaaatctgct tatttggcaa tgtgtctatc ctttagaaac 1620

agagttcttg tcgctatgga ttgggctaaa gtttatttct tgggtagaga ttcatctatc 1680

tag 1683

<210> 29
<211> 1638
<212> DNA
<213> Saccharomyces cerevisiae

<400> 29
atgctgccca gacttggttt tgcgaggact gctaggtcca tacaccgttt caagatgacc 60

cagatctcta aacctttttt ccattccact gaagttggta agcccggacc acagcagaag 120

ctatcgaaat cttacactgc ggtattcaag aaatggtttg tcagaggttt aaagttaacc 180

ttttacacga cgttggccgg cacattgtat gtgtcatacg agctgtacaa agaatcgaac 240

ccacccaaac aggttcccca atcgaccgct tttgctaatg gtttgaaaaa gaaggagctg 300

gttattttgg gtacaggctg gggcgccata tctcttttga agaaattaga cacgtctttg 360

tataacgtga ccgtggtgtc gccaagaagc ttctttttgt tcacaccgtt attaccctca 420

acgcctgtgg gtacgataga gatgaagtct attgtcgaac cggttagatc gatcgctaga 480

agaacgcctg gagaagttca ctacattgag gcggaagcgt tggacgttga tccaaaggcc 540

aaaaaagtaa tggtgcaatc ggtgtcagag gacgaatatt tcgtttcgag cttaagttac 600

gattatcttg ttgttagtgt aggcgctaaa accactactt ttaacattcc cggggtctat 660

ggcaatgcta acttcttgaa agagattgaa gatgctcaaa atattcgtat gaagttaatg 720

aaaaccatag aacaggcaag ttcatttcct gtgaacgatc cggaaaggaa gcgattatta 780

acgttcgtgg ttgttggagg gggccctacg ggggttgaat ttgccgccga actgcaagat 840

tacatcaatc aagatttgag gaagtggatg cccgacttaa gtaaagaaat gaaggttatc 900

ttaattgaag ccctgcctaa tatcctaaac atgttcgata agacgttgat caagtatgcc 960

gaggaccttt ttgccagaga tgaaattgac ttgcaagtga atactgccgt gaaagtcgta 1020

gagccaacct atatacgcac tctgcaaaac ggccaaacaa acacggatat cgaatacggg 1080

atgctggttt gggccacggg aaatgaacca atcgattttt caaagacact gatgagtaga 1140

ataccggagc aaactaatag gcgtggtctg ttaattaatg acaagttgga gcttctcggt 1200

tctgagaatt cgatttatgc aattggtgat tgtaccgcac acacgggttt ctttcccacg 1260

gcacaagttg cacatcagga aggcgaatac ttggccaaga tcttggataa aaaattacag 1320

atagaacaat tggaatggga catgctcaac agtaccgatg aaactgaggt atcacgtcta 1380

caaaaagagg ttaatttgag gaaatctaag ttggataagt tcaactacaa gcatatgggt 1440

gcccttgcgt acatcggctc tgaaaccgca attgcagatt tgcatatggg cgactcatca 1500

taccagttga aaggtatgtt tgccttcttg ttttggaaat ccgcttattt ggccatgtgt 1560

ctctctatca ggaataggat tttaattgcc atggactgga ccaaagttta ctttcttgga 1620

agggattcct ccgtgtag 1638

<210> 30
<211> 31
<212> DNA
<213> Artificial Sequence

<220>
<223> forward primer

<400> 30
cgagctcttc gcggccacct acgccgctat c 31

<210>    31
<211>    32
<212>    DNA
<213>    Artificial Sequence

<220>
<223>    reverse primer


<400>    31
gctctagata ttgatatagt gtttaagcga at                                    32


<210>    32
<211>    26
<212>    DNA
<213>    Artificial Sequence

<220>
<223>    forward primer


<400>    32
ggatccatgt ccgtaaagga actact                                           26


<210>    33
<211>    34
<212>    DNA
<213>    Artificial Sequence

<220>
<223>    reverse primer


<400>    33
acgcgtcgac ttaaaactgc aattcctttt gaat                                  34


<210>    34
<211>    26
<212>    DNA
<213>    Artificial Sequence

<220>
<223>    forward primer


<400>    34
gagctcaatt aaccctcact aaaggg                                           26


<210>    35
<211>    26
<212>    DNA
<213>    Artificial Sequence

<220>
<223>    reverse primer


<400>    35
gagctccaaa ttaaagcctt cgagcg                                           26

<210>    36
<211>    69
<212>    DNA
<213>    Artificial Sequence

<220>
<223>    forward primer


<400>    36
tgagcacgtg agtatacgtg attaagcaca caaaggcagc ttggagtatg gtgctgcaag    60

gcgattaag                                                            69


<210>    37
<211>    67
<212>    DNA
<213>    Artificial Sequence

<220>
<223>    reverse primer


<400>    37
aggcaagtgc acaaacaata cttaaataaa tactactcag taataacccg gctcgtatgt    60

tgtgtgg                                                              67


<210>    38
<211>    21
<212>    DNA
<213>    Artificial Sequence

<220>
<223>    forward primer


<400>    38
gccaaatgat ttagcattat c                                              21


<210>    39
<211>    21
<212>    DNA
<213>    Artificial Sequence

<220>
<223>    reverse primer


<400>    39
aaaaggagag ggccaagagg g                                              21


<210>    40
<211>    60
<212>    DNA
<213>    Artificial Sequence

<220>
<223>    forward primer


<400>    40
atgattagac aatcattaat gaaaacagtg tgggctaact ccagtcacga cgttgtaaaa         60

                                                                          60


<210>    41
<211>    60
<212>    DNA
<213>    Artificial Sequence

<220>
<223>    reverse primer


<400>    41
ctagatagat gaatctctac ccaagaaata aactttagcc aggtttcccg actggaaagc         60

                                                                          60


<210>    42
<211>    22
<212>    DNA
<213>    Artificial Sequence

<220>
<223>    forward primer


<400>    42
actgatcatc atttaaaaat gt                                                  22


<210>    43
<211>    22
<212>    DNA
<213>    Artificial Sequence

<220>
<223>    reverse primer


<400>    43
aaggaaaaaa attttcacac ta                                                  22


<210>    44
<211>    70
<212>    DNA
<213>    Artificial Sequence

<220>
<223>    forward primer


<400>    44
atgctgccca gacttggttt tgcgaggact gctaggtcca tacaccgttt ccagtcacga         60

cgttgtaaaa                                                              70

<210>    45
<211>    60
<212>    DNA
<213>    Artificial Sequence

<220>
<223>    reverse primer

<400>    45
ctacacggag gaatcccttc caagaaagta aactttggtc aggtttcccg actggaaagc      60

                                                                        60

<210>    46
<211>    20
<212>    DNA
<213>    Artificial Sequence

<220>
<223>    forward primer

<400>    46
caggaacata gtagaaagac                                                   20

<210>    47
<211>    18
<212>    DNA
<213>    Artificial Sequence

<220>
<223>    reverse primer

<400>    47
taacgcgaat cttccatg                                                     18

<210>    48
<211>    65
<212>    DNA
<213>    Artificial Sequence

<220>
<223>    forward primer

<400>    48
aatgtctcaa ccggttcaac gcgctgcagc acgctcattc cttcaaagag ctgcaccgcg      60

gtggc                                                                   65

<210>    49
<211>    65
<212>    DNA
<213>    Artificial Sequence

<220>
<223>    reverse primer


<400>    49
ttactgttta ccagtttttt ctttctcttt ccattcctta tctagagacc gggccccccg        60

ctagt                                                                    65


<210>    50
<211>    64
<212>    DNA
<213>    Artificial Sequence

<220>
<223>    forward primer


<400>    50
aatgtctaca tcatccgtac gttttgcatt taggcggttc tggcaagagc tgcaccgcgg        60

tggc                                                                     64


<210>    51
<211>    66
<212>    DNA
<213>    Artificial Sequence

<220>
<223>    reverse primer


<400>    51
ttatctgccc gtagtaattt cctttttgct ttctgcggcg ccgctgagac cgggcccccc        60

gctagt                                                                   66


<210>    52
<211>    19
<212>    DNA
<213>    Artificial Sequence

<220>
<223>    forward primer


<400>    52
ggacgtggcc tgtaaagtt                                                     19


<210>    53
<211>    23
<212>    DNA
<213>    Artificial Sequence

<220>
<223>    reverse primer

<400>    53
agaatatgca tgaacatatc cat                                              23


<210>    54
<211>    18
<212>    DNA
<213>    Artificial Sequence

<220>
<223>    forward primer


<400>    54
ctattgcgcg catgacta                                                    18


<210>    55
<211>    20
<212>    DNA
<213>    Artificial Sequence

<220>
<223>    reverse primer


<400>    55
tgcattgcct tctattatcc                                                  20

## Description

### BACKGROUND

### 1. Field

The present disclosure relates to yeast cells that produce a pyruvate-based metabolite from pyruvate in which the activity of a mitochondrial pyruvate carrier (MPC) is reduced compared to yeast cells that are not genetically engineered, and methods of producing a pyruvate-based metabolite by using the yeast cells.

### 2. Description of the Related Art

Microorganisms such as bacteria or yeasts can produce useful materials via a biological fermentation process. The biological fermentation process requires optimization of various intracellular metabolic pathways to produce a target material at a high yield.

Pyruvate is an end product of a glycolysis process within a cell. Pyruvate is a major substrate used in the tricarboxylic acid (TCA) cycle in mitochondria and is converted into a variety of metabolites. In some cases, pyruvate may be converted into metabolites within the TCA cycle or may be converted into lactate. In some other cases, pyruvate may be converted into diacetyl, acetoin, or butanediol via α-acetolactate. In still other cases, pyruvate may be converted into acetaldehyde, ethanol, or acetate via acetyl-CoA. In this regard, pyruvate is involved in the production of various intracellular metabolites, and accordingly, in order to maximize production of a target pyruvate-based metabolite by using a biotechnological method, there is a need to appropriately adjust one or more metabolic pathways in which pyruvate is involved, or adjust the availability of intracellular pyruvate.

Lactate, which is an example of the pyruvate-based metabolite, is an organic acid that is widely used in various industrial fields, such as those of food, pharmaceuticals, chemicals, and electronics. Lactate is non-toxic to the human body, and thus may be used as a flavor agent, a taste agent, or a preserving agent. Also, lactate is a raw material of polylactic acid (PLA) which is an environmentally-friendly biodegradable plastic (i.e. polymer material). In addition, lactate includes both a hydroxyl group and a carboxyl group, and thus is highly reactive. Accordingly, lactate may be easily converted into industrially important compounds, such as lactate ester, acetaldehyde, and propylene glycol, and thus has received attention as an alternative next generation chemical material in the chemical industry.

Lactate is currently produced by either a chemical synthesis process or a biochemical process. The chemical synthesis process is performed by oxidizing ethylene derived from crude oil, preparing lactonitrile through addition of hydrogen cyanide after acetaldehyde, purifying by distillation, and hydrolyzing by using hydrochloric acid or sulfuric acid. The biochemical process converts pyruvate to lactate via the enzyme lactate dehydrogenase. Also, lactate is produced commercially by incubating microorganisms such as bacteria and yeasts with an assimilable carbohydrate substrate, such as starch, sucrose, maltose, glucose, fructose, and xylose.

Also, ethanol, which is another example of a pyruvate-based metabolite, is a material that is converted from pyruvate by pyruvate decarboxylase (PDC) and alcohol dehydrogenase (ADH) within a cell, and such a material is produced commercially using a fermentation method for microorganisms such as yeasts that produce ethanol. Ethanol is used in various applications related to alcoholic beverages and fuels.

Therefore, a strain for efficiently producing a target pyruvate-based metabolite from pyruvate, and a method of producing a pyruvate-based metabolite using the strain have been demanded.

### SUMMARY

Provided are genetically engineered yeast cells that produce a pyruvate-based metabolite from pyruvate, wherein the genetically engineered yeast cell has a deletion or disruption mutation of a gene encoding MPC and the activity of a mitochondrial pyruvate carrier (MPC) protein is reduced in comparison with that of parent yeast cells not having a deletion or disruption mutation of the gene encoding MPC.

Also provided are methods of preparing the genetically engineered yeast cells, and methods of producing a pyruvate-based metabolite using the genetically engineered yeast cells.

Additional aspects will be set forth in part in the description which follows and, in part, will be apparent from the description, or may be learned by practice of the presented embodiments.

### BRIEF DESCRIPTION OF THE DRAWINGS

These and/or other aspects will become apparent and more readily appreciated from the following description of the embodiments, taken in conjunction with the accompanying drawings in which:

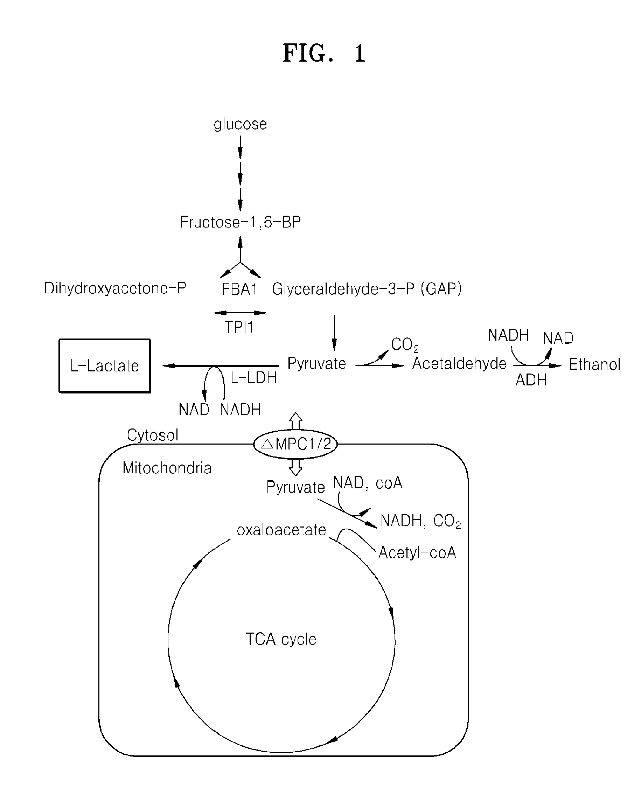

FIG. 1 is a diagram illustrating a lactate production pathway of a yeast cell having the ability to produce lactate;
FIG. 2 is a diagram of a p416-CCW12p-LDH vector;

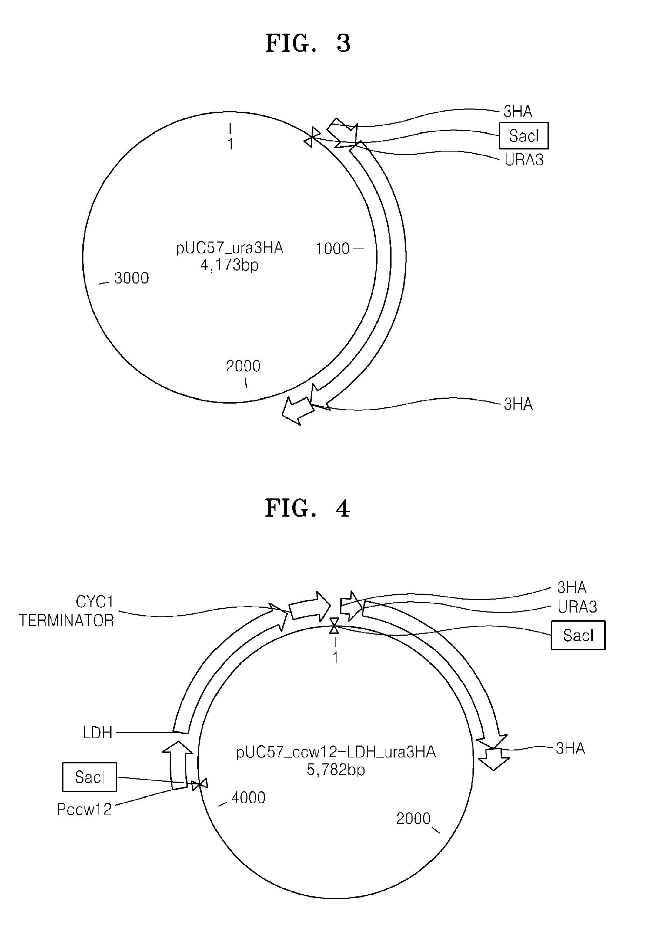

FIG. 3 is a diagram of a pUC57-ura3HA vector;
FIG. 4 is a diagram of a pUC57-CCW12-LDH-ura3HA vector;
FIG. 5 is a diagram of a pUC19-HIS3 vector;
FIG. 6 is a diagram of a pUC19-CCW12p-LDH-HIS3 vector; and
FIGS. 7A and 7B are each a graph showing culturing characteristics of strains of Δnde1Δnde2 and Δnde1Δnde2Δmpc1Δmpc2 under fermentation conditions.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

As used herein, the term "reduced activity" of a genetically engineered cell, enzyme or polypeptide denotes a genetically engineered cell, polypeptide or enzyme within a cell, or an isolated polypeptide or enzyme whose activity of a given type is lower than the activity measured in a comparable parent cell, polypeptide, or enzyme (e.g., the original or wild-type cell, polypeptide. or enzyme). The terms "activity reduces" or "reduced activity" of a cell, enzyme or polypeptide may also denote a cell, an isolated polypeptide or enzyme, or a polypeptide or enzyme within a cell having no activity of a given type. The activity of a cell, polypeptide, or enzyme may be reduced by any amount, e.g., about 20% or more, about 30% or more, about 40% or more, about 50% or more, about 55% or more, about 60% or more, about 70% or more, about 75% or more, about 80% or more, about 85% or more, about 90% or more, about 95% or more, or about 100% in comparison to the activity of a parent cell, wild-type polypeptide, or wild-type enzyme. The reduced activity of the cell (e.g., yeast), polypeptide, or enzyme may be confirmed using a commonly known method in the art. The reduction of the activity may be due to reduced expression of the gene, or reduced activity of the expressed polypeptide (e.g., a mutant polypeptide). The term "parent cell" denotes a cell not having a specific genetic modification resulting in a genetically engineered cell. The term "wild-type" polypeptide or polynucleotide denotes a polypeptide or polynucleotide not having a specific genetic modification resulting in a genetically engineered polypeptide or polynucleotide.

Activity of a cell, polypeptide, or enzyme may be reduced due to deletion or disruption of a gene encoding the polypeptide or enzyme (e.g., which a cell). As used herein, the "deletion" or "disruption" of the gene includes mutation or deletion of the gene or a regulatory region of the gene (e.g., operator, promoter or terminator regions of the gene), or a part thereof, sufficient to disrupt or delete gene function or the expression of a functional gene product. Mutations include substitutions, additions, and deletions of one or more bases in the gene or its regulator regions. As a result, the gene is not expressed or has a reduced amount of expression, or the activity of the encoded protein or enzyme is reduced or eliminated. The deletion or disruption of the gene may be accomplished by any suitable genetic engineering technique, such as homologous recombination, mutation induction, or molecular evolution. When a cell includes a plurality of copies of the same gene or at least two different polypeptide paralogs, at least one gene may be deleted or disrupted.

As used herein, the term "increased activity" of a genetically engineered cell, enzyme or polypeptide means that the genetically engineered cell, a polypeptide or enzyme within a cell, or an isolated polypeptide or enzyme exhibits an activity level of a given type that is higher than the activity level of a comparable parent cell, wild-type polypeptide, or wild-type enzyme. The activity of a cell, polypeptide, or enzyme may be increased by any amount, e.g., by about 5% or more, about 10% or more, about 15% or more, about 20% or more, about 30% or more, about 50% or more, about 60% or more, about 70% or more, or about 100% or more in comparison to the activity of a parent cell, wild-type polypeptide, or wild-type enzyme. A cell having increased activity of a cell, polypeptide, or enzyme may be confirmed by using any method commonly known in the art.

An increase in the activity of the cell, polypeptide, or enzyme may be caused by an increase in expression of the polypeptide or enzyme (e.g., within a cell) or an increase in a specific activity of the polypeptide or enzyme (e.g., within a cell). The increase in the expression may be caused by introduction of one or more polynucleotides encoding a polypeptide or enzyme into a cell, thereby introducing a new polynucleotide into the cell or providing an increased number of copies of an existing polynucleotide, by mutation of an expressed sequence to provide a mutant gene product having enhanced activity, or by mutation of a regulatory region of the polynucleotide so as to increase expression. Here, the introduced polynucleotide or the polynucleotide having the increased number of copies may be an endogenous gene or an exogenous gene. The endogenous gene may be a gene present in a genetic material contained within the microorganism. The exogenous gene may include a gene not normally occurring in the host cell that is integrated into the genetic material of the host cell (e.g., a chromosome of a host cell) or a gene introduced into the host cell by way of expression vector (e.g., a plasmid) or other construct.

As used herein, the gene manipulation and engineering may be performed by molecular biological methods known in the art (refer to Roslyn M. Bill, Recombinant Protein Production in Yeast: Methods and Protocols (2012), Sambrook et al., Molecular cloning, A laboratory manual: Cold Spring Harbor Laboratory (1989), R Daniel Gietz et al., Quick and easy yeast transformation using the LiAc/SS carrier DNA/PEG method: Nature protocols (2007)).

As used herein, the term "gene" denotes a nucleic acid fragment expressing a specific protein, and the fragment may include a coding region as well as non-coding regions or regulatory sequences (e.g., 5'-non coding sequences and a 3'-non coding sequences) in addition to the coding region.

As used herein, the term "pyruvate-based metabolite" or "pyruvate metabolite" denotes an intracellular metabolite converted from pyruvate. A pyruvate-based metabolite of the present disclosure may be a metabolite converted from pyruvate within a cytosol, or a metabolite of a pyruvate-based metabolite precursor that is converted from pyruvate in a cytosol. The pyruvate-based metabolite may include a metabolite directly converted from pyruvate by a biosynthetic step or a metabolite converted from the metabolite directly converted from pyruvate pyruvate by a biosynthetic step. The pyruvate-based metabolite may be at least one selected from lactate, ethanol, glycerol, acetate, formate, alanine, carbon dioxide, hydrogen, and a combination thereof. When the pyruvate-based metabolite is an organic acid, the pyruvate-based metabolite may include free organic acid, anionic form, or a salt thereof. Thus, free organic acid may be interchangeably used with its 'anionic form' or a salt thereof. For example, lactic acid may be interchangeably used with lactate or a salt thereof. The "pyruvate-based metabolite" may be secreted from the cell to the outside of the cell.

As used herein, the term "sequence identity" of a nucleic acid or polypeptide with respect to another nucleic acid or polypeptide refers to a degree of similarity in the sequence of bases or amino acid residues of two sequences (or a given region of two sequences) that are aligned to best match each other for comparison. The sequence identity is a value obtained via optimal alignment and comparison of the two sequences in the specific region for comparison, in which a partial sequence in the specific region for comparison may be added or deleted with respect to a reference sequence. The sequence identity represented in a percentage may be calculated by, for example, comparing two sequences that are aligned to best match each other in the specific region for comparison, determining matched sites, wherein the same amino acid or base in the two sequences is found, to obtain the number of the matched sites, dividing the number of the matched sites in the two sequences by a total number of sites in the compared specific regions (i.e., the size of the compared region), and multiplying a result of the division by 100 to obtain a sequence identity as a percentage. The sequence identity as a percentage may be determined using a known sequence comparison program, for example, BLASTN or BLASTP (NCBI), CLC Main Workbench (CLC bio), MegAlign^{™}(DNASTAR Inc), or the like.

In identifying a polypeptide or polynucleotide with the same or similar function or activity with respect to various types of species, various levels of sequence identity may be applied. In some embodiments, the polypeptide or polynucleotide may have an amino acid sequence, or a nucleic acid sequence, respectively, with a sequence identity of, for example, 50% or greater, 55% or greater, 60% or greater, 65% or greater, 70% or greater, 75% or greater, 80% or greater, 85% or greater, 90% or greater, 95% or greater, 96% or greater, 97% or greater, 98% or greater, 99% or greater, or 100%, with respect to the other polypeptide or polynucleotide to which it was compared.

According to one aspect of the present disclosure, provided is a genetically engineered yeast cell that produces a pyruvate-based metabolite from pyruvate, wherein the genetically engineered yeast cell has a deletion or disruption mutation of a gene encoding MPC and the activity of a mitochondrial pyruvate carrier (MPC) is reduced compared to a parent yeast cell not having a deletion or disruption mutation of the gene encoding MPC.

According to another aspect of the present disclosure, there is provided a method of producing a pyruvate-based metabolite from pyruvate using a yeast cell by reducing the activity of one or more MPC proteins in the yeast cell.

Production of the pyruvate-based metabolite from pyruvate may occur in a cytosol.

In regard to the production of the pyruvate-based metabolite converted from pyruvate or a precursor of the pyruvate-based metabolite in a cytosol using a yeast cell, when the level of pyruvate in a cytosol is increased, an amount of the pyruvate-based metabolite produced from pyruvate or the pyruvate-based metabolite precursor may be increased. The level of pyruvate within a cytosol may be increased by the reduction of the activity of the MPC polypeptides.

In general, pyruvate is produced from glucose through glycolysis in the cytosol, and the produced pyruvate is delivered to a mitochondrion by one or more MPC proteins. The transported pyruvate is then converted into acetyl-CoA, and used to produce metabolites or energy via a tricarboxylic acid (TCA) cycle. When the activity of MPC is reduced in a cell and the delivery of pyruvate from the cytosol to the mitochondrion is inhibited, a pyruvate pool within the cytosol may be enhanced, and accordingly the production of the pyruvate-based metabolite may be increased therefrom.

The activity of the MPC may be reduced by deletion or disruption of the MPC-coding polynucleotide. The MPC is a polypeptide present in the inner mitochondrial membrane and acts to transport pyruvate from the cytosol to mitochondria. The MPC may be an MPC 1, an MPC2, an MPC3, and any one of these proteins, or a combination thereof, may be inhibited by deletion or disruption of the corresponding gene. For example, the MPC may have an amino acid sequence with a sequence identity of about 60% or greater, about 70% or greater, about 80% or greater, about 90% or greater, about 95% or greater, about 96% or greater, about 97% or greater, about 98% or greater, or about 99% or greater with respect to SEQ ID NOs: 1, 2, or 3. In greater detail, the MPC may have an amino acid sequence with a sequence identity of about 95% or greater with respect to SEQ ID NOs: 1, 2, or 3. The MPC 1, MPC2, and MPC3 may each have an amino acid sequence of SEQ ID NOs: 1, 2, and 3, respectively. The MPC-coding polynucleotide may be a polynucleotide encoding a protein having an amino acid sequence with a sequence identity of about 95% or greater with respect to SEQ ID NOs: 1, 2, or 3. For example, the MPC-coding polynucleotide may have a nucleotide sequence of SEQ ID NOs: 4, 5, or 6. The MPC-coding polynucleotide having nucleotide sequences of SEQ ID NOs: 4, 5, and 6 each encode a MPC1, a MPC2, and a MPC3, respectively.

The yeast cell may belong to the *Saccharomyces* genus, *Candida* genus, *Schizosaccharomyces* genus, *Kluyveromyces* genus, *Pichia* genus, *Issachenkia* genus, or *Hansenula* genus. In some embodiments, the yeast cell may belong to *Saccharomyces* genus, *Candida* genus, *Kluyveromyces* genus, or *Issachenkia* genus. The *Saccharomyces* genus may be, for example, *S*. *cerevisiae, S. bayanus, S. boulardii, S. bulderi, S. cariocanus, S. cariocus, S. chevalieri, S. dairenensis, S. ellipsoideus, S. eubayanus, S. exiguus, S. florentinus, S. kluyveri, S. martiniae, S. monacensis, S. norbensis, S. paradoxus, S. pastorianus, S. spencerorum, S. turicensis, S. unisporus, S. uvarum,* or *S*. *zonatus.*

The genetically engineered yeast cell of the present disclosure may have an increased ability (e.g., increased efficiency) to produce a pyruvate-based metabolite. The pyruvate-based metabolite may be at least one selected from lactate, ethanol, glycerol, acetate, formate, alanine, carbon dioxide, hydrogen, and a combination thereof. In some embodiments, the conversion of a pyruvate-based metabolite from pyruvate or from the pyruvate-based metabolite precursor may occur in the cytosol. The increased level of pyruvate in the cytosol due to the reduced activity of the MPC may facilitate the conversion of the pyruvate-based metabolite from pyruvate.

The genetically engineered yeast cell may be produced from a strain that produces a pyruvate-based metabolite naturally, whereby the genetic engineering described herein enhances the production of the metabolite. Alternatively, the genetically engineered yeast cell may be produced from a strain that does not produce a pyruvate-based metabolite naturally. When the yeast cell may not produce a pyruvate-based metabolite naturally (e.g., when the yeast cell does not contain the endogenous genes necessary to produce the specific pyruvate-based metabolite), the genes involved in the production of the pyruvate-based metabolite may be introduced into the yeast cell to produce the pyruvate-based metabolite. In either case, the yeast cell in which activity of the MPC is reduced may increase the production of the pyruvate-based metabolite in comparison to the cell that is not genetically engineered to reduce activity of the MPC.

In some embodiments, the yeast cell may have an ability to produce lactate. The yeast cell may include a polypeptide converting pyruvate into lactate, such as a lactate dehydrogenase (LDH). In some other embodiments, the yeast cell may be genetically engineered to have an increased activity of the polypeptide converting pyruvate into lactate as compared to a parent cell. The LDH may be an NAD(P)-dependent enzyme or a stereo-specific enzyme. The stereo-specific enzyme may produce L-lactate, D-lactate, or a combination thereof. The NAD(P)-dependent enzyme may be an enzyme that is classified into EC 1.1.1.27 that functions on L-lactate or EC 1.1.1.28 that functions on D-lactate.

The yeast cell having an ability to produce lactate may be genetically engineered to have increased LDH activity as compared to a parent cell. The yeast cell used herein may include at least one LDH gene, and the LDH gene may be an endogenous gene or an exogenous gene. The polynucleotide encoding the LDH may be an enzyme derived from bacteria, yeasts, fungi, mammals or reptiles. The polynucleotide may be a polynucleotide that encodes at least one LDH from at least one of *Lactobacillus helveticus, L.bulgaricus, L.johnsonii, L.plantarum, Pelodiscus sinensis japonicus, Ornithorhynchus anatinus, Tursiops truncatus, Rattus norvegicus,* and *Xenopus laevis.* An LDH from *Pelodiscus sinensis japonicus,* an LDH from *Ornithorhynchus anatinus,* an LDH from *Tursiops truncatus,* and an LDH from *Rattus norvegicus* may each have an amino acid sequence with a sequence identity of about 60% or greater, about 70% or greater, about 80% or greater, about 90% or greater, about 95% or greater, about 96% or greater, about 97% or greater, about 98% or greater, or about 99% or greater with respect to SEQ ID NOs: 7, 8, 9, and 10. In some embodiments, the LDH-coding polynucleotide may be a polynucleotide encoding an amino acid sequence with a sequence identity of about 95% or greater with respect to SEQ ID NOs: 7, 8, 9, and 10. In some other embodiments, the LDH-coding polynucleotide may be a polynucleotide having a nucleotide sequence with a sequence identity of about 95% or greater with respect to SEQ ID NO: 11.

The LDH-coding polynucleotide may be contained in a vector, and the vector may include a replication origin, a promoter, an LDH-coding polynucleotide, and a terminator. The replication origin may include a yeast autonomous replication sequence (ARS). The yeast ARS may be stabilized by a yeast centrometric sequence (CEN). The promoter may be selected from a cytochrome c (CYC) promoter, a transcription elongation factor (TEF) promoter, a glycerol-3-phosphate dehydrogenase (GPD) promoter, and an alcohol dehydrogenase (ADH) promoter. The CYC promoter, the TEF promoter, the GPD promoter, and the ADH promoter may each have a nucleotide sequence of SEQ ID NOs: 13, 14, 15, and 16.

The terminator may be selected from a terminator of a gene encoding a phosphoglycerate kinase 1 (PGK1), a terminator of a gene encoding a cytochrome c 1 (CYC1), and a terminator of a gene encoding a galactokinase 1 (GAL1). The CYC1 terminator may have a nucleotide sequence of SEQ ID NO: 17.

The vector may further include a selection marker. The selection marker may any known selection marker in the art. For example, the selection marker may be auxotrophic marker, or antibiotic resistance selection marker.

The LDH-coding polynucleotide may be included in a genome in a specific location of a yeast cell. The specific location of the yeast cell may include a locus of a gene to be deleted and disrupted, such as MPC, pyruvate decarboxylase (PDC) and cytochrome-c oxidoreductase 2 (CYB2).

The yeast cell may include a polynucleotide that encodes one LDH or a polynucleotide that encodes multiple LDH copies, e.g., 2 to 10 copies. The yeast cell may include a polynucleotide that encodes multiple LDH copies into, for example, 1 to 8, 1 to 7, 1 to 6, 1 to 5, 1 to 4, or 1 to 3 copies. When the yeast cell includes the polynucleotide encoding multiple LDHs, each polynucleotide may include copies of the same LDH polynucleotide or copies of polynucleotides encoding at least two different LDHs. The multiple copies of the polynucleotide encoding exogenous LDHs may be included in the same locus or multiple loci in a genome of a host cell, and the promoter or terminator of each copy of the polynucleotide may be identical to or different from each other.

In order to optimize the production of the pyruvate-based metabolite, a metabolic pathway of the yeast cell may be optimized. That is, activity of a metabolic pathway which is a competitive pathway with the production of the pyruvate-based metabolite may be reduced, or activity of a metabolic pathway which is a synergistic pathway with the production of the pyruvate-based metabolite may be increased.

In some embodiments, the yeast cell used herein may exhibit lactate productivity and may further include genetic modification to optimize lactate production. For example, in the yeast cell, activity of a polypeptide that converts pyruvate into acetaldehyde, a polypeptide that converts lactate into pyruvate, or a polypeptide that converts dihydroxyacetone phosphate (DHAP) into glycerol-3-phosphate, or a combination thereof may be reduced.

In the genetically engineered yeast cell, a gene that encodes the polypeptide that converts pyruvate into acetaldehyde may be deleted or disrupted. The polypeptide that converts pyruvate into acetaldehyde may be an enzyme that is classified as EC 4.1.1.1. In some embodiments, the enzyme may be a pyruvate decarboxylase (PDC), for example, a PDC1. The polypeptide that converts pyruvate to acetaldehyde may have an amino acid sequence with a sequence identity of about 60% or greater, about 70% or greater, about 80% or greater, about 90% or greater, about 95% or greater, about 96% or greater, about 97% or greater, about 98% or greater, or about 99% or greater with respect to SEQ ID NO: 18. The gene that encodes the polypeptide that converts pyruvate to acetaldehyde may be a polynucleotide encoding an amino acid sequence with a sequence identity of about 95% or greater with respect to SEQ ID NO: 18, or may have a nucleotide sequence of SEQ ID NO: 19.

In the yeast cell, a gene that encodes the polypeptide that converts lactate into pyruvate may be disrupted. The polypeptide that converts lactate into pyruvate may be a CYC-dependent enzyme. The polypeptide that converts lactate into pyruvate may be an enzyme that is classified as EC 1.1.2.4 that acts on D-lactate or EC 1.1.2.3 that acts on L-lactate. The polypeptide that converts lactate into pyruvate may be lactate cytochrome c-oxidoreductase, a CYB2 (CAA86721.1), a CYB2A, a CYB2B, or a DLD1. The polypeptide that converts lactate into pyruvate may have an amino acid sequence with a sequence identity of about 60% or greater, about 70% or greater, about 80% or greater, about 90% or greater, about 95% or greater, about 96% or greater, about 97% or greater, about 98% or greater, or about 99% or greater with respect to SEQ ID NO: 20. The gene that encodes the polypeptide that converts lactate into pyruvate may be a polynucleotide encoding an amino acid sequence with a sequence identity of about 95% or greater with respect to SEQ ID NO: 20, or may have a nucleotide sequence of SEQ ID NO: 21.

In the yeast cell, a gene that encodes the polypeptide that converts DHAP into glycerol-3-phosphate may be disrupted. The polypeptide may be glycerol-3-phosphate dehydrogenase (GPD), which is an enzyme that catalyzes reduction of DHAP to glycerol-3-phosphate by using oxidation of NADH or NADP to NAD+ or NADP+. The polypeptide may belong to EC 1.1.1.8, and the cytosolic GPD may be GPD1. The cytosolic GPD may have an amino acid sequence with a sequence identity of about 60% or greater, about 70% or greater, about 80% or greater, about 90% or greater, about 95% or greater, about 96% or greater, about 97% or greater, about 98% or greater, or about 99% or greater with respect to SEQ ID NO: 22. The gene that encodes cytosolic GPD may a polynucleotide encoding an amino acid sequence with a sequence identity of about 95% or greater with respect to SEQ ID NO: 22, or may have a nucleotide sequence of SEQ ID NO: 23.

In some embodiments, in the yeast cell, the polynucleotide that encodes the MPC, the polynucleotide that encodes the polypeptide converting pyruvate into acetaldehyde, the polynucleotide that encodes the polypeptide converting lactate into pyruvate, or the polynucleotide that encodes the polypeptide converting DHAP into glycerol-3-phosphate, or a combination thereof may be disrupted, and in addition, a polynucleotide that encodes a polypeptide converting pyruvate into lactate may be included or further introduced into the yeast cell. Here, the yeast cell may be *Saccharomyces cerevisiae.*

In some embodiments, the pyruvate-based metabolite may include ethanol, and thus the genetically engineered yeast cell may produce ethanol. In the yeast cell that may produce ethanol, activity of converting pyruvate into ethanol may be increased. In addition, the yeast producing ethanol may include PDC, which is an enzyme catalyzing conversion from pyruvate into acetaldehyde, and alcohol dehydrogenase (ADH), which is an enzyme catalyzing conversion from acetaldehyde into ethanol.

The PDC may be an enzyme that is classified as EC 4.1.1.1, and the enzyme may be a PDC1. The PDC may have an amino acid sequence with a sequence identity of about 60% or greater, about 70% or greater, about 80% or greater, about 90% or greater, about 95% or greater, about 96% or greater, about 97% or greater, about 98% or greater, or about 99% or greater with respect to SEQ ID NO: 18. The gene that encodes the PDC may be a polynucleotide encoding an amino acid sequence with a sequence identity of about 95% or greater with respect to SEQ ID NO: 18, or may have a nucleotide sequence of SEQ ID NO: 19.

The ADH may be an enzyme that is classified as EC 1.1.1.1, and the enzyme may be an ADH1. The ADH may have an amino acid sequence with a sequence identity of about 60% or greater, about 70% or greater, about 80% or greater, about 90% or greater, about 95% or greater, about 96% or greater, about 97% or greater, about 98% or greater, or about 99% or greater with respect to SEQ ID NO: 24. The gene that encodes the ADH may be a polynucleotide encoding an amino acid sequence with a sequence identity of about 95% or greater with respect to SEQ ID NO: 24, or may have a nucleotide sequence of SEQ ID NO: 25.

In some embodiments, the yeast cell used herein may have an ability to produce ethanol and may further include genetic modification to optimize the production of ethanol. For example, in the yeast cell, the activity of a polypeptide that converts pyruvate into lactate, a polypeptide that converts DHAP into glycerol-3-phosphate, or a combination thereof may be reduced. The polypeptide may be the same as described above.

In some other embodiments, in the yeast cell, the polynucleotide that encodes the MPC, the polynucleotide that encodes the polypeptide converting pyruvate into lactate, or the polynucleotide that encodes the polypeptide converting DHAP into glycerol-3-phosphate, or a combination thereof may be disrupted, and in addition, a polynucleotide that encodes a polypeptide converting pyruvate into ethanol may be included or further introduced into the yeast cell. Here, the yeast cell may be *Saccharomyces cerevisiae.*

In some embodiments, the pyruvate-based metabolite may include glycerol, acetate, formate, alanine, carbon dioxide, or hydrogen, and may be produced by using a known biosynthetic pathway or enzyme in the art (Appl Environ Microbiol. 2009 April; 75(7): 1867-875; Indian Journal of Microbiology, Volume 48, Issue 2, pp 252-266; Biotechnol Adv. 2001 Jun;19(3):201-23).

In some embodiments, the yeast cell in which the activity of the MPC is reduced may be a yeast cell in which activity of a mitochondrial NADH dehydrogenase is further reduced. The external mitochondrial NADH dehydrogenase may be an enzyme that is classified as EC. 1.6.5.9 or EC. 1.6.5.3. The NADH dehydrogenase may be a type II NADH: ubiquinone oxidoreductase. The NADH dehydrogenase may be located on the outer surface of the inner mitochondrial membrane facing a cytoplasm. The NADH dehydrogenase may be an enzyme catalyzing oxidation of cytosolic NADH to NAD+. The NADH dehydrogenase may re-oxidize cytosolic NADH formed by a glycolysis process. The NADH dehydrogenase may provide cytosolic NADH to a mitochondrial respiratory chain. The NADH dehydrogenase may be Nde1 or Nde2, or a combination thereof. The NADH dehydrogenase may be distinguished from an internal mitochondrial NADH dehydrogenase NDE1 that is present and functions inside mitochondria. The NADH dehydrogenase may have an amino acid sequence with a sequence identity of about 60% or greater, about 70% or greater, about 80% or greater, about 90% or greater, about 95% or greater, about 96% or greater, about 97% or greater, about 98% or greater, or about 99% or greater with respect to SEQ ID NOs: 26 or 27. For example, NDE1 and NDE2 may each have amino acid sequences of SEQ ID NO: 26 and SEQ ID NO: 27.

The genes encoding the external mitochondrial NADH dehydrogenase may each have an amino acid sequence with a sequence identity of about 95% or greater with respect to SEQ ID NOs: 26 and 27, or may have a polynucleotide sequence of SEQ ID NOs: 28 or 29.

The method of producing a pyruvate-based metabolite comprises culturing the yeast cell, and collecting the pyruvate-based metabolite from the yeast culture.

The culturing of the yeast cell may be performed in a medium containing an assimilable-carbon source, for example, glucose. The medium used in the culturing of the yeast cell may be a common medium appropriate for growth of a host cell, a minimum medium or a composite medium containing an appropriate supplement.

The medium used in the culturing process may be a medium that may satisfy requirements of a particular yeast cell. The medium may be one selected from a carbon source, a nitrogen source, salts, trace elements, and a combination thereof.

Culture conditions may be appropriately controlled in the genetically engineered yeast cell to obtain a pyruvate-based metabolite. The culturing may be conducted in conditions suitable for the production of a pyruvate-based metabolite. The yeast cell may be cultured under aerobic conditions, preferably microaerobic conditions or anaerobic conditions. In some embodiments, the yeast cell may be cultured under aerobic conditions for its proliferation, and then under microaerobic conditions to produce a pyruvate-based metabolite. As used herein, the term "anaerobic conditions" refers to an environment devoid of oxygen. As used herein, the term "microaerobic conditions" when used in reference to a culture or growth condition is intended to mean that the dissolved oxygen concentration in the medium remains between 0 and about 10% of saturation for dissolved oxygen in liquid media. Microaerobic conditions also includes growing or resting cells in liquid medium or on solid agar inside a sealed chamber maintained with an atmosphere of less than 1% oxygen. The percent of oxygen can be maintained by, for example, sparging the culture with an N₂/CO₂ mixture or other suitable non-oxygen gas or gases. The oxygen conditions may include maintaining a dissolved oxygen (DO) concentration of 0% to 10%, for example, 0%, 0 to 8%, 0 to 6%, 0 to 4%, 0 to 2%, 1 to 10%, 1 to 8%, 1 to 6%, 1 to 4%, or 1 to 2%, 2 to 10%, 2 to 8%, 2 to 6%, 2 to 4%, 3 to 10%, 3 to 8%, 3 to 6%, 4 to 10%, 4 to 8%, or 4 to 6% of saturation for dissolved oxygen in liquid media. Fermented broth may be controlled to maintain its pH in a range of about 2 to about 7.

The yeast cell used herein may be incubated by methods of continuous culture, semicontinuous culture, batch culture, or fed batch culture, or a combination thereof.

As used herein, the term "culture condition" denotes a condition for culturing a yeast cell. The culture condition may be, for example, a carbon source, a nitrogen source, or oxygen conditions for the yeast cell. The carbon source that is used by the yeast cell includes monosaccharides, disaccharides, or polysaccharides. In particular, glucose, fructose, mannose, or galactose may be used. The nitrogen source that is used by the yeast cell may be an organic nitrogen compound or an inorganic nitrogen compound. In particular, amino acid, amide, amine, nitrate, or ammonium salt may be used. A metabolic pathway may be corrected with a carbon source or a nitrogen source that may be actually used by a yeast cell.

The collecting of the pyruvate-based metabolite from the yeast culture may further include a process of isolating or separating the pyruvate-based metabolite from the yeast culture. Separation of the pyruvate-based metabolite from the culture may be performed by a separation method commonly known in the art. The separation method may be centrifugation, filtration, extraction, distillation, ion-exchange chromatography, or crystallization.

Hereinafter, the present disclosure will be described in further detail with reference to the following examples. These examples are for illustrative purposes only and are not intended to limit the scope of the invention.

### Example 1. Preparation of strain for highly-efficient production of lactate and preparation of expression vector

In order to block a production pathway of ethanol and glycerol as main byproducts by using *Saccharomyces cerevisiae CEN.PK2-1D* (*MATaura3-52; trp1-289; leu2-3,112; his3*Δ*1; MAL2-8^{c}; SUC2,* EUROSCARF accession number: 30000B) as a lactate production strain, the following genes were disrupted: a pyruvate decarboxylase (pdc1) gene, which is a main enzyme of alcohol fermentation; a NAD-dependent glycerol-3-phosphate dehydrogenase (gpd1) gene, which is a main enzyme of glycerol biosynthesis; and a L-lactate cytochrome-c oxidoreductase 2 (cyb2) gene, which is a lactate degrading enzyme. A lactate dehydroxygenase gene was introduced into each position of the disrupted genes of *Saccharomyces cerevisiae CEN.PK2-1D,* thereby obtaining *Saccharomyces cerevisiae CEN.PK2-1D* Δpdc1::ldh Δcyb2::ldhΔgpd1::ldh (KCTC 12415BP).

### 1.1 Preparation of a L-LDH overexpression vector

A CCW12 promoter polymerase chain reaction (PCR) fragment obtained by performing a PCR with a genomic DNA of *Saccharomyces cerevisiae CEN.PK2-1D* as a template and using primers of SEQ ID NO: 30 and SEQ ID NO: 31 was digested with SacI and XbaI, and the resultant was inserted into a p416-GPD vector (refer to http://www.atcc.org/products/all/87360.aspx) digested with Sacl and Xbal, thereby producing a p416-CCW12p vector suitable for overexpression of L-ldh.

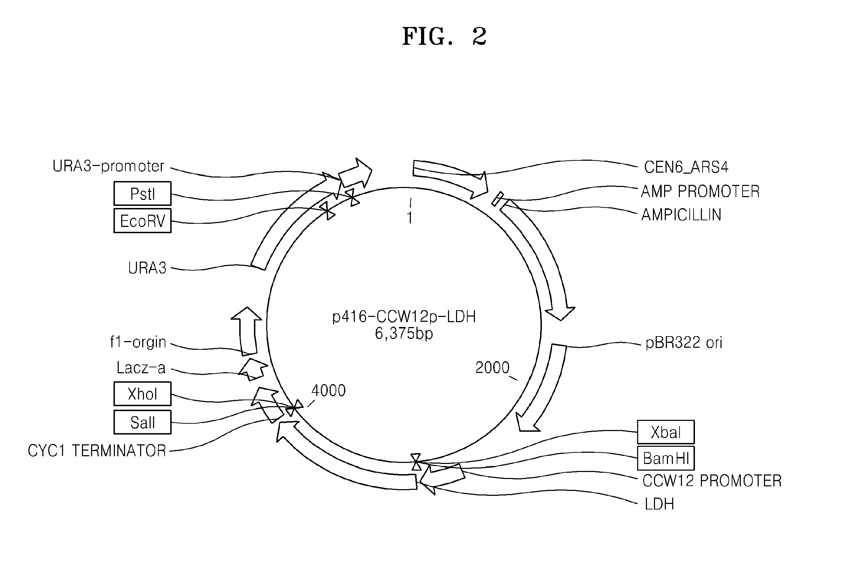

Then, L-ldh gene (SEQ ID NO: 11) was amplified from *Pelodiscus sinensis japonicus* genomic DNA by PCR using primers of SEQ ID NO: 32 and SEQ ID NO: 33. The resulting L-ldh PCR fragment and a p416-CCW12p obtained therefrom were digested with BamHI and SalI, and ligated, thereby producing p416-CCW12p-LDH, which is a L-ldh expression vector. The L-ldh expression vector also has a yeast autonomous replication sequence/a yeast centrometric sequence of SEQ ID NO: 12, a CYC promoter of SEQ ID NO: 13, a GPD promoter of SEQ ID NO: 15, and a CYC1 terminator of SEQ ID NO: 17. The L-ldh expression vector included a polynucleotide encoding L-ldh of SEQ ID NO: 11. FIG. 2 is a view illustrating a p416-CCW12p-LDH vector.

### 1.2 Preparation of introduction vector for introducing L-ldh gene into genome

In order to increase lactate production by redox balance enhancement or glycolysis pathway engineering, an L-ldh gene may be additionally introduced to a genome of a KCTC12415BP strain. In order to introduce the L-ldh gene to the genome of the KCTC12415BP strain, a gene introduction vector may be prepared as follows.

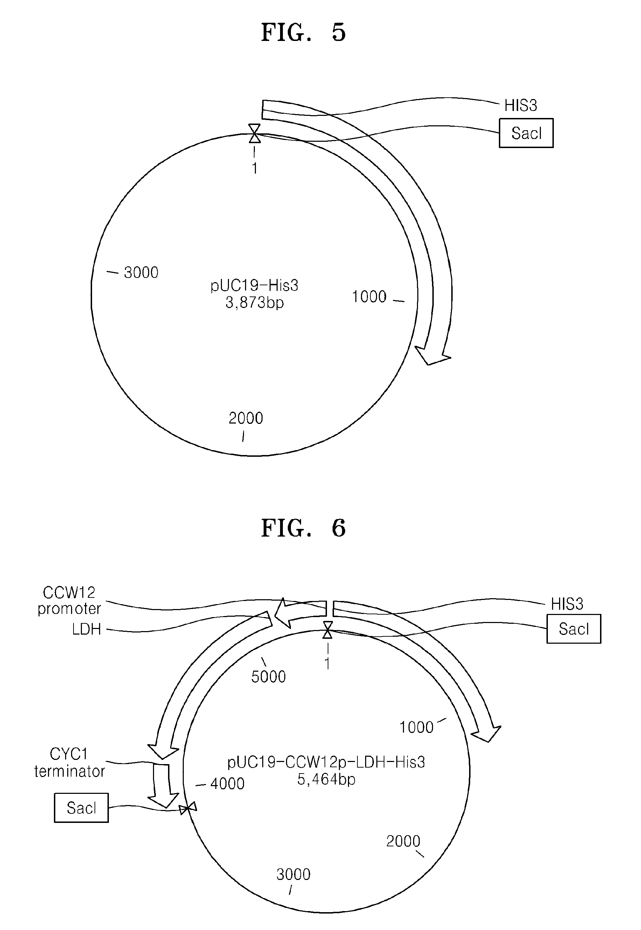

PCR was performed by using the prepared p416-CCW12p-LDH as a template with primers of SEQ ID NOs: 34 and 35. The resulting PCR fragment and the prepared pUC19-HIS3 vector (refer to Appl Environ Microbiol. 2002 May;68(5):2095-100, and FIG. 5) were cleaved by SacI, and the resultant was ligated to prepare pUC19-CCW12p-LDH-HIS3 (see FIG. 6). PCR was performed by using the prepared pUC19-CCW12p-LDH-HIS3 as a template with primers of SEQ ID NOs: 36 and 37, and thus a cassette to be inserted in a location of TRP1 was prepared. The cassette including L-ldh may be inserted to a TRP1 gene, and in this case, L-ldh may be inserted as the TRP1 gene is deleted. A L-ldh inserted strain may be prepared as follows.

A strain of KCTC12415BP was spread onto a YPD agar plate (including 10g of yeast extract, 20 g of peptone, and 20 g of glucose) and incubated for 24 hours at 30 °C, and then a colony obtained therefrom was inoculated in about 10 ml of a YPD liquid medium and cultured for 18 hours at 30 °C. The sufficiently grown culture solution was inoculated in about 50 ml of a YPD liquid medium contained in a 250 ml-flask at a concentration of 1 % (v/v) and in an incubator vortexing at a rate of about 230 rpm and at 30 °C.

After about 4 to 5 hours, when the optical density at 600 nanometers (OD₆₀₀) reached about 0.5, the culture was centrifuged at a rate of about 4,500 rpm for about 10 minutes to harvest cells, and the cells were suspended in a lithium acetate solution at a concentration of about 100 mM. Then, the suspended cells were centrifuged at a rate of about 4,500 rpm for about 10 minutes to harvest cells, and the harvested cells were re-suspended in a lithium acetate solution at a concentration of about 1 M including about 15% of glycerol.

In order to delete a TRP1 gene and express L-ldh at the same time, a L-ldh expression cassette was mixed with 50% of polyethylene glycol and a single stranded carrier DNA, 100 µl of the re-suspension solution was added thereto, and reacted in a water tub at 42 °C for 1 hour. Then, the culture solution was spread on a histidine (his)-free minimal agar plate (including YSD, 6.7 g/L of yeast nitrogen base without amino acids, and 1.4 g/L of amino acid dropout mix (-his)) and grown at 30 °C for 24 hours or more. Ten colonies (i.e., mutant strains) grown on the his-free minimal agar plate were selected, spread onto a fresh YSD (-his) agar plate, and at the same time, inoculated into a YSD (-his) liquid medium to isolate the genomic DNA from the mutant strains above by using a commonly used kit (Gentra Puregene Cell kit, Qiagen, USA). In order to confirm deletion of the TRP1 gene, a PCR was performed using the isolated genomic DNA of the mutant strain as a template with primers of SEQ ID NOs: 38 and 39, and then, electrophoresis was performed on the obtained PCR product to confirm insertion of the L-ldh expression cassette. As a result, the obtained strain was Δtrp1::ldh (KCTC12415BP Δtrp1::ldh).

### Example 2. Preparation of Saccharomyces cerevisiae strain from which nde1 and nde2 are deleted

### 2.1 Preparation of a nde1 gene deletion cassette

Preparation of pUC57-CCW12p-LDH-ura3HA: a PCR was performed using the p416-CCW12p-LDH of Example 1 as a template with primers of SEQ ID NO: 34 and SEQ ID NO: 35. The resulting PCR fragment and the prepared pUC57-ura3HA vector (Genetics 116: 541-545, August, 1987) were digested with SacI and ligated, thereby producing pUC57-CCW12p-LDH-ura3HA.

A PCR was performed using the prepared pUC57-CCW12p-LDH-ura3HA as a template with primers of SEQ ID NO: 40 and SEQ ID NO: 41 to prepare an nde1 gene deletion cassette.

### 2.2 Preparation of Saccharomyces cerevisiae strain from which nde1 is deleted

A mutant strain of the Δtrp1::ldh strain (KCTC12415BPAtrp1::ldh), in which nde1 is deleted, was prepared in the same manner as follows.

The Δtrp1::ldh strain (KCTC12415BPΔtrp1::ldh) was spread onto a YPD agar plate (including 10 g of yeast extract, 20 g of peptone, and 20 g of glucose) and incubated for 24 hours at 30°C, and then, a colony obtained therefrom was inoculated in about 10 ml of a YPD liquid medium and cultured for 18 hours at 30°C. The sufficiently grown culture solution was inoculated in about 50 ml of a YPD liquid medium contained in a 250 ml-flask at a concentration of 1 % (v/v) and in an incubator vortexing at a rate of about 230 rpm and at 30°C. After about 4 to 5 hours, when the OD₆₀₀ reached about 0.5, the culture was centrifuged at a rate of about 4,500 rpm for about 10 minutes to harvest cells, and the cells were resuspended in a lithium acetate solution at a concentration of about 100 mM. Then, the suspended cells were centrifuged at a rate of about 4,500 rpm for about 10 minutes to harvest cells, and the harvested cells were re-suspended in a lithium acetate solution at a concentration of about 1 M including about 15% of glycerol.

In order to delete an nde1 gene, the nde 1 deletion cassette was mixed with 50% of polyethylene glycol and single stranded carrier DNA, 100 µl of the re-suspension solution containing the water-soluble competent cells was added thereto, and reacted in a water tub for about 1 hour at 42 °C. Then, the culture solution was spread on a uracil-free minimal agar plate (including YSD, 6.7g/L of yeast nitrogen base without amino acids, and 1.4g/L of amino acid dropout mix (-ura)) and grown for about 24 hours or more at 30 °C. Ten colonies grown on the plate were selected, spread onto the fresh uracil-free minimal agar plate, and at the same time, inoculated into a liquid medium including the same components contained in the uracil-free minimal agar plate to isolate the genomic DNA from the above mutant strains by using a commonly used kit (Gentra Puregene Cell kit, Qiagen, USA). In order to confirm deletion of the nde 1 gene, a PCR was performed using the isolated genomic DNA of the mutant strain as a template with primers of SEQ ID NOs: 42 and 43, and then, electrophoresis was performed on the obtained PCR product to confirm nde1 deletion. As a result, the obtained strain was KCTC12415BPΔtrp1::ldhΔnde1+ura3.

Also, for additional gene deletion using the gene deletion vector, a selection marker URA 3 gene, which was used for the deletion of the nde1 gene, was removed by using a URA3 pop-out method. That is, KCTC12415BPΔtrp1::ldhΔnde1+ura3 was inoculated in about 10 ml of a YPD liquid medium, cultured for about 18 hours at 30 °C, spread on a 5-FOA agar plate (including YSD, 6.7g/L of yeast nitrogen base without amino acids, 1.4g/L of amino acid dropout mix, and 1µg/L of 5-fluoroorotic acid), and cultured for about 24 hours at 30 °C. Ten colonies (URA3 pop-out strain) grown on the 5-FOA agar plate were selected, spread onto the fresh 5-FOA agar plate, and at the same time, cultured in a YPD liquid medium to isolate genomic DNA from the selected strain by using a commonly used kit (Gentra Puregene Cell kit, Qiagen, USA). In order to confirm deletion of the URA 3 gene by using the genomic DNA of the isolated URA3 pop-out strain as a template, a PCR was performed using primers of SEQ ID NOs: 42 and 43, and then electrophoresis was performed on the obtained PCR product to conform URA3 gene deletion. As a result, Δnde1 strain (KCTC12415BP Δtrp1::ldhΔnde1) was obtained.

### 2.3. Preparation of nde2 gene deletion cassette

A vector for disrupting a nde 1 gene, i.e., pUC57-CCW12p-LDH-ura3HA prepared in Example 2.1 was used to delete a nde2 gene by using a homologous recombination method. In order to prepare a nde2 gene deletion cassette, PCR was performed using the prepared pUC57-CCW12p-LDH-ura3HA as a template with primers of SEQ ID NOs: 44 and 45.

### 2.4. Preparation of S. cerevisiae strain from which nde1 and nde2 are deleted

An nde2 deleted strain of Δnde1 (KCTC12415BPΔtrp1::ldhΔnde1) was prepared in the same manner as follows.

The Δnde1 strain was treated with a lithium acetate solution in the same manner as in Example 2.2, thereby obtaining competent cells.

In order to delete an nde2 gene, the nde2 deletion cassette prepared in Example 2.3 was mixed with 50% of polyethylene glycol and a single stranded carrier DNA, and 100 µl of the re-suspension solution for the competent cells was added thereto to obtain colonies on a uracil-free minimal agar plate in the same manner as in Example 2.2. Ten colonies (i.e., mutant strains) grown on the uracil-free minimal agar plate were selected, spread onto a fresh uracil-free minimal agar plate, and at the same time, inoculated into a liquid medium including the same components as those contained in the uracil-free minimal agar plate to isolate the genomic DNA from the mutant strains above by using a commonly used kit (Gentra Puregene Cell kit, Qiagen, USA). In order to confirm nde 2 gene deletion, a PCR was performed using the isolated genomic DNA of the mutant strain as a template with primers of SEQ ID NOs: 46 and 47, and then, electrophoresis was performed on the obtained PCR product to confirm nde2 gene deletion. As a result, the obtained strain was referred to as KCTC12415BPΔtrp1::ldhΔnde1Δnde2+ura3.

Also, for additional gene deletion, a selection marker URA3 gene, which was used for nde2 gene deletion, was removed from *Saccharomyces cerevisiae* KCTC12415BP (Δtrp1::ldhΔnde1Δnde2+ura3) by using a URA3 pop-out method in the same manner as in Example 2.2. In order to confirm URA 3 gene deletion by using the genomic DNA of the isolated URA3 pop-out strain as a template, a PCR was performed using primers of SEQ ID NOs: 46 and 47, and then, electrophoresis was performed on the obtained PCR product to confirm URA3 gene deletion. As a result, the obtained strain was Δnde1Δnde2 (KCTC12415BPΔtrp1::ldhΔnde1Δnde2).

### Example 3. Preparation of Saccharomyces cerevisiae from which mpc1 is deleted

### 3.1. Preparation of mpc1 gene deletion cassette

In order to delete a mpc1 gene by using a homologous recombination method, PCR was performed using the pUC57-ura3HA of Example 2 as a template with primers of SEQ ID NOs: 48 and 49 (i.e., mpc1_del_F and mpc1_del_R) to prepare a mpc1 gene deletion cassette.

### 3.2. Preparation of Saccharomyces cerevisiae from which mpc1 is deleted

A mpc1 deleted strain of the Δnde1Δnde2 (KCTC12415BPΔtrp1::ldh Δnde1Δnde2) was prepared in the same manner as follows.

The Δnde1Δnde2 strain of Example 2 was treated with a lithium acetate solution in the same manner as in Example 2.2, thereby obtaining re-suspension solution of competent cells.

In order to delete a mpc1 gene, the mpc 1 gene deletion cassette prepared in Example 3.1 was mixed with 50% of polyethylene glycol and single stranded carrier DNA, 100 µl of the re-suspension solution of competent cells was added thereto to obtain colonies on a uracil-free minimal agar plate in the same manner as in Example 2.2. Ten colonies (i.e., mutant strains) grown on the uracil-free minimal agar plate were selected, spread onto a fresh uracil-free minimal agar plate, and at the same time, inoculated into a liquid medium including the same components as those contained in the uracil-free minimal agar plate to isolate the genomic DNA from the mutant strains above by using a commonly used kit (Gentra Puregene Cell kit, Qiagen, USA). In order to confirm mpc1 gene deletion, a PCR was performed using the isolated genomic DNA of the mutant strain as a template with primers of SEQ ID NOs: 52 and 53 (i.e., mpc1_up_F and mpc1_down_R), and then, electrophoresis was performed on the obtained PCR product to confirm mpc1 gene deletion. As a result, the obtained strain was KCTC12415BPΔtrp1::ldh Δnde1Δnde2Δmpc1+ura3.

Also, for additional gene deletion, a selection marker URA3 gene, which was used for the mpc1 gene deletion, was removed from *Saccharomyces cerevisiae* KCTC12415BP (Δtrp1::ldhΔnde1Δnde2 Δmpc1+ura3) by using a URA3 pop-out method in the same manner as in Example 2.2. In order to confirm URA3 deletion by using the genomic DNA of the isolated URA3 pop-out strain as a template, a PCR was performed using primers of SEQ ID NOs: 52 and 53, and then, electrophoresis was performed on the obtained PCR product to confirm URA3 gene deletion. As a result, the obtained strain was Δmpc1 (KCTC12415BPΔtrp1::ldhΔnde1Δnde2Δmpc1).

### Example 4. Preparation of Saccharomyces cerevisiae strain from which mpc2 is deleted

### 4.1. Preparation of mpc2 gene deletion cassette

In order to delete an mpc2 gene by using a homologous recombination method, a PCR was performed using the pUC57-ura3HA of Example 2 as a template with primers of SEQ ID NOs: 50 and 51 (i.e., mpc2_del_F and mpc2_del_R) to prepare an mpc2 gene deletion cassette.

### 4.2. Preparation of Saccharomyces cerevisiae strain from which mpc2 is deleted

An mpc2 deleted strain of the Δnde1Δnde2 (KCTC12415BPΔtrp1::ldhΔnde1Δnde2) was prepared in the same manner as follows.

The Δnde1Δnde2 strain was treated with a lithium acetate solution in the same manner as in Example 2.2, thereby obtaining a re-suspension solution of competent cells.

In order to delete an mpc2 gene, the mpc2 gene deletion cassette prepared in Example 4.1 was mixed with 50% of polyethylene glycol and single stranded carrier DNA, 100 µl of the re-suspension solution of competent cells was added thereto to obtain colonies on a uracil-free minimal agar plate in the same manner as in Example 2.2. Ten colonies (i.e., mutant strains) grown on the uracil-free minimal agar plate were selected, spread onto a fresh uracil-free minimal agar plate, and at the same time, inoculated into a liquid medium including the same components as those contained in the uracil-free minimal agar plate to isolate the genomic DNA from the mutant strains above by using a commonly used kit (Gentra Puregene Cell kit, Qiagen, USA). In order to confirm mpc2 gene deletion, a PCR was performed using the isolated genomic DNA of the mutant strain as a template with primers of SEQ ID NOs: 54 and 55 (i.e., mpc2_up_F and mpc2_down_R), and then, electrophoresis was performed on the obtained PCR product to confirm mpc2 gene deletion. As a result, the obtained strain was KCTC12415BPΔtrp1::ldh Δnde1Δnde2Δmpc2+ura3.

Also, for additional gene deletion, a selection marker URA3 gene, which was used for the mpc2 gene deletion, was removed from *Saccharomyces cerevisiae* KCTC12415BP (Δtrp1::ldhΔnde1Δnde2Δmpc2+ura3) by using a URA3 pop-out method in the same manner as in Example 2.2. In order to confirm URA3 deletion by using the genomic DNA of the isolated URA3 pop-out strain as a template, a PCR was performed using primers of SEQ ID NOs: 54 and 55, and then, electrophoresis was performed on the obtained PCR product to confirm URA3 gene deletion. As a result, the obtained strain was referred to as Δmpc2 (KCTC12415BPΔtrp1::ldh Δnde1Δnde2Δmpc2).

### Example 5. Preparation of Saccharomyces cerevisiae strain from which mpc1 and mpc2 are deleted

In order to prepare a mutant of Δmpc1Δmpc2 from which a mpc1 gene and a mpc2 gene are deleted as follows, the Δmpc1 strain (KCTC12415BPΔtrp1::ldhΔnde1Δnde2Δmpc1) of Example 3.2 was treated with a lithium acetate solution in the same manner as in Example 2.2, thereby obtaining competent cells.

In order to delete an mpc2 gene, the mpc2 deletion cassette prepared in Example 4.1 was mixed with 50% of polyethylene glycol and single stranded carrier DNA, and 100 µl of the re-suspension solution of competent cells was added thereto to obtain colonies on a uracil-free minimal agar plate in the same manner as in Example 2.2. Ten colonies (i.e., mutant strains) grown on the uracil-free minimal agar plate were selected, spread onto a fresh uracil-free minimal agar plate, and at the same time, inoculated into a liquid medium including the same components as those contained in the uracil-free minimal agar plate to isolate the genomic DNA from the mutant strains above by using a commonly used kit (Gentra Puregene Cell kit, Qiagen, USA). In order to confirm mpc2 gene deletion, a PCR was performed using the isolated genomic DNA of the mutant strain as a template with primers of SEQ ID NOs: 54 and 55 (i.e., mpc2_up_F and mpc2_down_R), and then, electrophoresis was performed on the obtained PCR product to confirm mpc2 gene deletion. As a result, the obtained strain was referred to as KCTC12415BPΔtrp1::ldh Δnde1Δnde2Δmpc1Δmpc2+ura3.

Also, a selective marker URA 3 gene was removed from KCTC12415BPΔtrp1::ldhΔnde1Δnde2Δmpc1Δmpc2+ura3 by using a URA3 pop-out in the same manner as in Example 2.2. In order to confirm URA 3 gene deletion by using the genomic DNA of the isolated URA3 pop-out strain as a template, a PCR was performed using primers of SEQ ID NOs: 54 and 55, and then, electrophoresis was performed on the obtained PCR product to confirm URA3 gene deletion. As a result, the obtained strain was Δmpc1Δmpc2 (KCTC12415BPΔtrp1::ldh Δnde1Δnde2Δmpc1Δmpc2).

### Example 6. Production of ethanol and lactate using each of a strain in which a MPC gene is disrupted

Each of the strains Δnde1Δnde2, Δmpcl, Δmpc2, and Δmpc1Δmpc2 that were prepared in Examples 2 to 5, respectively, was spread onto a YPD agar plate and cultured at 30 °C for 24 hours or more, and then a colony obtained therefrom was inoculated in 50 ml of a YPD liquid medium including 40 g/L of glucose and cultured at 30 °C for 16 hours under aerobic conditions. Here, the fermentation was performed as follows. An amount of the culture that has a cell concentration of 5.0 at a light absorbance (optical density) of 600 nm (OD 5.0) as measured by using a spectrophotometer in the 50 ml of the culture medium was quantified and centrifuged, and the supernatant was removed. Then, the cells were re-suspended and re-inoculated in 50 ml of a new YPD liquid medium including 80 g/L of glucose, and then incubated.

The cells were microaerobically cultured in a sealed flask at a temperature of 30°C for 24 hours or more, while stirring at a rate of about 90 rpm. During the incubation, samples were periodically obtained from the flask, and the obtained samples were centrifuged at a rate of about 13,000 rpm for about 10 minutes. Then, concentrations of ethanol of the supernatant, lactate, and glucose were analyzed by using high-performance liquid chromatography (HPLC).

As shown in Table 1, L-lactate production of the Δmpc1 strain was increased from 31.0 g/L to 35.1 g/L compared to the Δnde1Δnde2 strain used as a reference strain, and ethanol production of the Δmpc1 strain was increased from 19.7 g/L to 23.4 g/L compared to the Δnde1Δnde2 strain used as a reference strain. In addition, L-lactate production of the Δmpc1Δmpc2 strain was increased from 31.0 g/L to 35.6 g/L compared to the Δnde1Δnde2 strain used as a reference strain, and ethanol production of the Δmpc1Δmpc2 strain was increased from 19.7 g/L to 23.2 g/L compared to the Δnde1Δnde2 strain used as a reference strain. As a result, it was confirmed that the strains from which the MPC gene was deleted had increased productivities and increased yields of lactate and ethanol.

**[Table 1]**

| **Strain** | **Characteristic** | **OD** | **LA (g/L)** | **Yield (%)** | **EtOH (g/L)** | **Yield (%)** | **LA+EtO H (g/L)** |
|---|---|---|---|---|---|---|---|
| **Δnde1Δ nde2** | **KCTC12415BPΔ trp1::ldhΔnde1Δ nde2** | **18.1** | **31.0** | **46.8** | **19.7** | **42.9** | **50.7** |
| Δmpc1 | KCTC12415BPΔt rp1::ldhΔnde1Δn de2Δmpc1 | 16.2 | 35.1 | 46.2 | 23.4 | 45.3 | 58.5 |
| Δmpc2 | KCTC12415BPΔt rp1::ldhΔnde1Δn de2Δmpc2 | 18.9 | 33.1 | 43.8 | 24.2 | 49.4 | 57.3 |
| Δmpc1Δ mpc2 | KCTC12415BPΔt rp1::ldhΔnde1Δn de2Δmpc1Δmpc2 | 21.6 | 35.6 | 46.7 | 23.2 | 43.2 | 58.8 |

In regard to Table 1 above, the strains were cultured in a 50 ml flask for about 30 hours. Here, LA denotes lactate and EtOH denotes ethanol.

### Example 7. Production of ethanol and lactate using Δmpc1Δmpc2 strain

The Δmpc1Δmpc2 strain prepared in Example 5 was spread on a YPD agar plate and cultured at 30 °C for 24 hours or more, and then a colony obtained therefrom was inoculated in 100 ml of a YPD liquid medium including 80 g/L of glucose and cultured at 30 °C for 16 hours under aerobic conditions. Here, 100 ml of the strain culture solution was separately inoculated in a bioreactor containing 1 L of a synthetic medium and cultured.

The culture was performed with initial concentrations of 60 g/L of glucose and 20 g/L of a yeast extract at 30 °C. During the culture, pH was maintained at about pH 5 for up to 16 hours, at about pH 4.5 for up to 24 hours, and at about pH 3.0 for up to 60 hours by using 5N Ca(OH)₂, and a concentration of the glucose was maintained at 20 g/L. Additional synthetic medium compositions include 50 g/L of K₂HPO₄, 10 g/L of MgSO₄, 0.1 g/L of tryptophan, and 0.1 g/L of histidine in addition to the glucose.

A cell concentration in the culture solution was measured by using a spectrophotometer. During the culture, samples were periodically obtained from a bioreactor, and the obtained samples were centrifuged at a rate of 13,000 rpm for 10 minutes. Then, concentrations of metabolites of the supernatant, lactate, and glucose were analyzed by using HPLC.

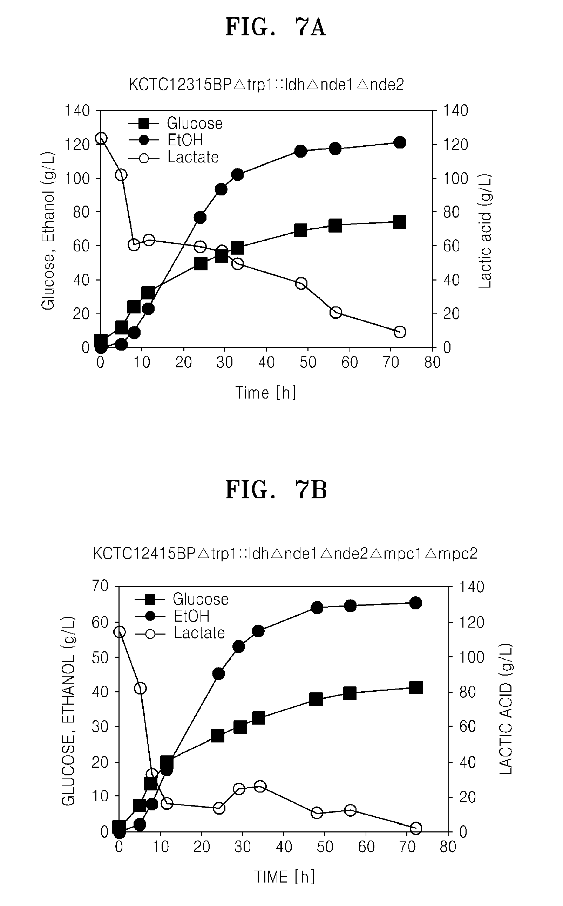

As shown in FIG. 7, the Δmpc1Δmpc2 strain (FIG. 7B) exhibited excellent lactate productivity, and a lactate yield thereof also was increased compared to the Δnde1Δnde2 strain (FIG. 7A) used as a reference strain. The lactate production of the Δmpc1Δmpc2 strain was increased from 115.8 g/L to 128.2 g/L and the yield thereof was increased from 57.7% to 63.8% compared to the Δnde1Δnde2 strain used as a reference strain. Also, the Δmpc1Δmpc2 strain exhibited increased ethanol productivity, and an ethanol yield thereof also was increased compared to the Δnde1Δnde2 strain as a reference strain. The ethanol production of the Δmpc1Δmpc2 strain was increased from 34.5 g/L to 37.8 g/L and the yield thereof was increased from 16.4% to 18.2% compared to the Δnde1Δnde2 strain used as a reference strain.

### [Accession Number]

Research Center Name: Korean Collection for Type Cultures (KCTC)
Accession Number: KCTC12415BP
Accession Date : 20130530

As described above, according to the one or more of the above embodiments of the present invention, a yeast cell may increase a level of pyruvate within a cytosol by reducing activity of a MPC, and accordingly a yeast cell may produce a pyruvate-based metabolite, which is converted from pyruvate, at a high concentration and a high yield. Also, according to the one or more of the above embodiments of the present invention, a method of producing a pyruvate-based metabolite may produce a pyruvate-based metabolite at a high concentration and a high yield.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A genetically engineered yeast cell that produces a pyruvate-based metabolite from pyruvate,
wherein the yeast cell has a deletion or disruption mutation of a gene encoding a mitochondrial pyruvate carrier (MPC) and the activity of the MPC is reduced compared to that of a parent yeast cell not having a deletion or disruption mutation of the gene encoding MPC.

2. The yeast cell of claim 1, wherein the yeast cell belongs to the genus *Saccharomyces,* the genus *Candida,* the genus *Schizosaccharomyces,* the genus *Kluyveromyces,* the genus *Pichia,* the genus *Issachenkia,* or the genus *Hansenula.*

3. The yeast cell of claim 1 or 2, wherein the MPC is MPC1, MPC2 or MPC3, or a combination thereof; preferably wherein the MPC comprises a polypeptide having an amino acid sequence with a sequence identity of 95% or greater with respect to an amino acid sequence selected from SEQ ID NO: 1, SEQ ID NO: 2, and SEQ ID NO: 3, or wherein the gene encoding the MPC has at least one nucleic acid sequence selected from SEQ ID NO: 4, SEQ ID NO: 5, and SEQ ID NO: 6.

4. The yeast cell of any one of claims 1 to 3, wherein the pyruvate-based metabolite is at least one selected from lactate, ethanol, glycerol, acetate, formate, alanine, carbon dioxide, and hydrogen.

5. The yeast cell of claim 4, wherein the pyruvate-based metabolite is lactate.

6. The yeast cell of claim 5, wherein the yeast cell comprises a polypeptide that converts pyruvate into lactate and the activity of the polypeptide is increased as compared to a parent yeast cell.

7. The yeast cell of claim 5, wherein (a) the yeast cell comprises a polynucleotide encoding lactate dehydrogenase; and/or (b) the yeast cell exhibits reduced activity of converting pyruvate into acetaldehyde, converting lactate into pyruvate, and/or converting dihydroxyacetone phosphate (DHAP) into glycerol-3-phosphate; and/or (c) the yeast cell contains a deletion or disruption mutation of a gene encoding a polypeptide for converting pyruvate into acetaldehyde, a gene encoding a polypeptide for converting lactate into pyruvate or a gene encoding a polypeptide for converting DHAP into glycerol-3-phosphate, or a combination thereof.

8. The yeast cell of claim 6, wherein the polypeptide converting pyruvate into lactate has an amino acid sequence with a sequence identity of 95% or greater with respect to SEQ ID NO: 7; and/or wherein the polynucleotide encoding the polypeptide converting pyruvate into lactate has a nucleotide sequence of SEQ ID NO: 11.

9. The yeast cell of claim 4, wherein the pyruvate-based metabolite is ethanol.

10. The yeast cell of claim 9, wherein (a) the yeast cell exhibits increased activity of converting pyruvate into ethanol as compared to a parent yeast cell; and/or (b) the yeast cell comprises pyruvate decarboxylase (PDC) or alcohol dehydrogenase (ADH), or a combination thereof; and/or (c) the yeast cell exhibits reduced activity of converting pyruvate into lactate or converting DHAP into glycerol-3-phosphate, or a combination thereof, as compared to a parent yeast cell.

11. The yeast cell of any one of claims 1 to 10, wherein the yeast cell exhibits reduced mitochondrial NADH dehydrogenase activity as compared to a parent yeast cell.

12. The yeast cell of claim 11, wherein the mitochondrial NADH dehydrogenase is NDE1, NDE2 or NDE3, or a combination thereof; preferably wherein the mitochondrial NADH dehydrogenase has an amino acid sequence with a sequence identity of 95% or greater with respect to SEQ ID NO: 26 or SEQ ID NO: 27.

13. A method of producing a pyruvate-based metabolite, the method comprising:
culturing the yeast cell of any one of claims 1 to 12; and
collecting the pyruvate-based metabolite from the culture.

14. The method of claim 13, wherein the pyruvate-based metabolite is at least one selected from lactate, ethanol, glycerol, acetate, formate, alanine, carbon dioxide, and hydrogen; and/or wherein the culturing is performed under microaerobic conditions.

15. A method of preparing a yeast cell that produces a pyruvate metabolite from pyruvate, the method comprising disrupting or deleting one or more genes encoding a mitochondrial pyruvate carrier (MPC) protein in a yeast cell.

16. A method of increasing the production efficiency of a pyruvate metabolite from pyruvate, the method comprising disrupting or deleting one or more genes encoding a mitochondrial pyruvate carrier (MPC) protein in a pyruvate metabolite-producing yeast cell, whereby the production efficiency of the pyruvate metabolite is increased.

17. The method of claim 15 or 16, wherein the method comprises disrupting or deleting one or more genes encoding MPC1, MPC2 or MPC3, or combination thereof.
